# EUROPEAN PATENT APPLICATION

(11) **EP 1 980 269 A1**
(43) Date of publication of application: **15.10.2008**
(21) Application number: 07106164.2
(22) Date of filing: 13.04.2007
(51) Int. Cl.: A61K 39/395, A61P 31/04, C07K 16/12, C07K 14/31

(54) **Prevention of staphylococcus biofilm formation**

(71) Applicant: KATHOLIEKE UNIVERSITEIT LEUVEN, 3000 Leuven (BE)
(72) Inventor: The designation of the inventor has not yet been filed
(74) Representative: van Loon, C.J.J.

(57) **Abstract**

The present application discloses antibodies directed to protein SE2232 of *Staphylococcus epidermis*, and homologous proteins which are useful in preventing biofilm formation by said micro-organism. The antibodies can be used to coat medical devices which are inserted or implanted into the mammalian, preferably human body. Further these antibodies or vaccines comprising said proteins can be used to vaccinate subjects to prevent or treat natural occurring biofilm formation during infection.

## Description

The invention relates to the field of medical microbiology, more specifically the prevention of biofilm formation by bacteria, more specifically wherein said bacteria are *Staphylococcus genus* bacteria.

### BACKGROUND OF THE INVENTION

*Staphylococcus epidermis* and other coagulase-negative staphylococci (CoNS) are the leading cause of foreign-body infections (FBI), i.e. infections which occur on devices which have been inserted or implanted in the body, such as (but not limited to) intravascular devices (stents), prosthetic valves, endovascular grafts, orthopaedic endoprotheses and cerebrovascular shunts. For prosthetic valve endocarditis, 40-50% of the infections are due to *S*. *epidermis,* while this bacterial species is responsible for 50-70% of catheter-related infections (van Eiff, C. et al., 2001, Postgrad. Med. 110(4):63-76). In the United States alone, it is estimated that infections caused by *S*. *epidermidis* cost the public health system at least $1 billion/year (Yao, Y. et al., 2005, J. Infect. Dis. 191:289-298).
The presence of foreign bodies facilitates the transition of *S*. *epidermidis* from a commensal microorganism to an opportunistic pathogen (Gotz, F. and Peters, G., 2000, In: Waldvogel, F.A. and Bisno, A.L. (ed.) Infections associated with indwelling medical devices. ASM Press, Washington DC). Biofilm formation on the surface of foreign bodies is a key factor in this process (Christensen, G.D. et al., 1982, Infect. immune. 37:318-326).
The development of a biofilm occurs in several steps. Initial attachment can be based on direct binding to the abiotic surface of an implant. This process is mediated by several proteins and carbohydrate factors (e.g. staphylococcal surface proteins SSP-1, SSP-2 (Veenstra, G.J. et al., 1996, J. Bacteriol. 178:537-541), capsular polysaccharide adhesin (PS/A) (Mack, D., 1999, J. Hosp. infect. 43(Suppl.):S113-S125; Shiro, H. et al., J. Infect. Dis. 169:1042-1049)). Alternatively, indirect binding to the surface of implanted medical devices coated with an absorbed layer of blood plasma proteins such as fibrinogen, fibronectin, collagen, vitronectin and elastin can be mediated via cell-wall-associated (CWA) proteins SdrG (Fbe) (Davis, S.L. et al., 2001, J. Biol. Chem. 276:27799-27805; Nilsson, M. et al., 1998, Infect. immune. 66:2666-2673), Embp (Williams, R.J. et al., 2002, Infect. immune. 70:6805-6810), GehD (Bowden, m.G. et al., 2002, J. Biol. Chem. 277:43017-43023), AtlE (Heilmann, C. et al., 1997, Mol. Microbiol. 24:1013-1024) and Ebps (Park, P.W. et al., 1996, J. Biol. Chem. 271:15803-15809), respectively.
In a second phase, the bacteria proliferate and accumulate into multilayered cell clusters that are embedded in an extracellular material. Bacterial proliferation and production of extracellular matrix molecules lead to a more structured biofilm.
The *ica*ADBC operon mediates the cell clustering and synthesis of the staphylococcal polysaccharide intercellular adhesin (PIA) (von Eiff, c. et al., 2002, Lancet Infect. Dis. 2:677). PIA facilitates intercellular adhesion and plays an essential role in cell accumulation. PIA has been identified as an important virulence factor of *S. epidermidis* (Rupp, M.E. et al., 1999, Infect. Immun. 67:2627-2632; Rupp, M.E. et al., 1999, Infect.Immun. 67:2656-2659). Proteins also seem to be essential for accumulation. The *S*. *aureus* Bap homologue protein, (Bhp) has been proposed to promote primary attachment to abiotic surfaces, as well as intercellular adhesion during biofilm formation (Cucarella, C. et al., 2001, J. Bacterial. 183:2888-2896). The accumulation-associated protein (Aap) is thought to play an essential role in cell accumulation, independent of PIA (Hussain, M. et al., 1997, Infect. Immun. 65:519-524). It has been shown that polyclonal and monoclonal antibodies against Aap can significantly reduce *S*. *epidermidis* accumulation (Hussain, m et al., *supra*; Sun, D. et al., 2005, Clin. Diagn. Lab. Immunol. 12:93-100). Detachment of cell clusters from an existing biofilm can occur, and this is attributed to the expression of a class of peptides known as phenol-soluble modulins (Yao, Y. et al., *supra,* Otto, M. et al., 2004, J. Infect. Dis. 190:748-755). However, our understanding of the mechanism of the detachment process is very limited.
Because most infections due to caogulase-negative staphylococci are nocosomial, it is not surprising that these infections have become increasingly resistant to multiple antibiotics: 80-90% of the isolated strains shows inducible beta-lactamase activity, 80% are methicillin-resistant (von Eiff, C. et al., 2001, Postgrad. Med. 110:63-76). Further, bacteria within the biofilm are tolerant to antibiotics and antimicrobial agents. It is estimated that sessile bacteria in biofilms are 1,000 to 1,500 times more resistant to antibiotics than their planktonic counterparts (Costerton, J., 1999, J. Antimicrob. Agents 11:217-221). This antibiotic resistance of biofilms often leads to the failure of conventional antibiotic therapy and necessitates removal and reinsertion of the infected device (Stewart, P.S. and Costerton, J.W., 2001 Lancet 358:135-138; Hoyle, B.D. and Costerton, J.W., 1991, Prog. Drug Res. 37:91-105, von Eiff, C. et al., Lancet Infect. Dis. 2:677-685 and Vuong, C. and Otto, M, 2002, Microbes Infect. 4:481-489).

The traditional approach to prevent biofilm formation in vivo is local or systemic administration of bactericidal agents (Danese, P.N., 2002, Chem. Biol. 9:873-880). Targeting specific staphylococcal biofilm-associated virulence factors, such as the formation of a slime capsule, is a novel approach for the treatment of staphylococcal infections (Vuong, C. and Otto, M, *supra*). Antibodies against extracellular macromolecules and surface binding proteins essential for cell-surface and cell-cell interaction adhesion, such as PIA, teichoic acids, Fbe and Aap have been shown to prevent biofilm formation without killing the bacteria (e.g. Bowden, M.G. et al., 2005, Microbiology 151:1453-1464).

Yet, there still exists need for better or alternative therapy for preventing formation of biofilms.

### SUMMARY OF THE INVENTION

The invention now relates to an antibody against protein SE2232 of *Staphylococcus epidermis* and antibodies against proteins from *Staphylococcus* spp. which are homologous with said SE2232 protein. Said antibodies are preferably selected from the group consisting of polyclonal antibodies, monoclonal antibodies, single chain antibodies, humanised antibodies and SE2232 binding antibody fragments such as F(ab), F(ab)2 and scFV.

The invention further comprises a method for preventing biofilm formation by *Staphylococcus* on a medical device comprising coating said medical device with an antibody according to the invention. Said medical device is preferably an intravascular device such as a stent or a cannula, a prosthesis, such as an orthopaedic endoprothesis or a prosthetic valve, a pacemaker, a catheter, a drain, an endovascular graft or a cerebrovascular shunt. This medical device is to be implanted or inserted into a mammalian body, preferably a human body.

A further embodiment of the invention is the use of an antibody according of the invention for preventing biofilm formation, preferably wherein the antibody is coated onto a medical device. Also comprised within the invention is a method to prevent biofilm formation in a mammal *in vivo* and the use of the antibodies of the invention for such a method.

### DESCRIPTION OF THE FIGURES

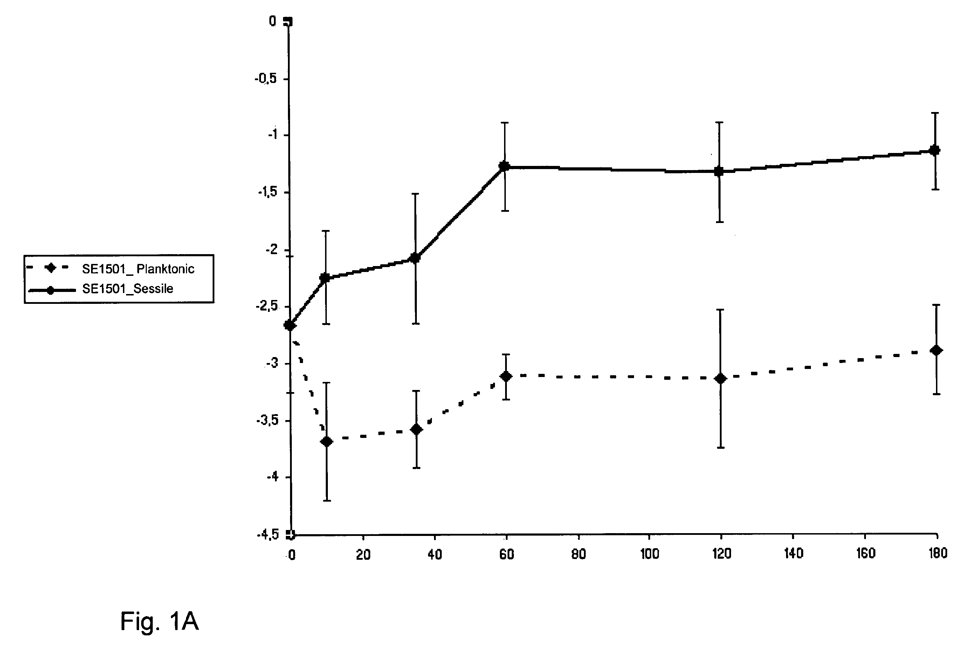

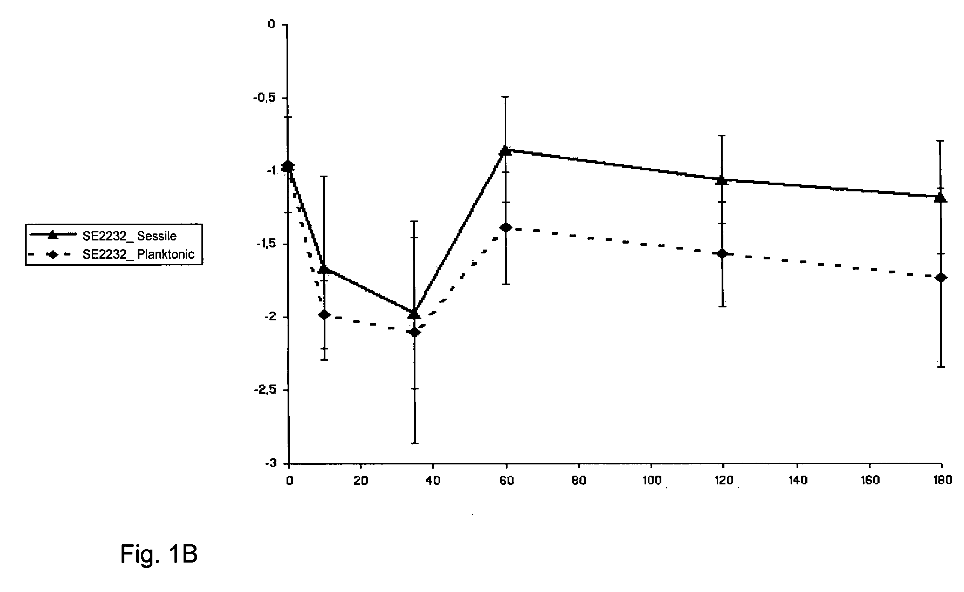

**FIG. 1**. In vitro expression of genes SE1501 and SE2232 in sessile versus planktonic bacteria after inoculation in 0.9% NaCl. Bacteria in end-exponential growth phase were suspended in 0.9% NaCl with catheter fragments at time 0 min. Gene expression is quantified as the log10 cDNA/gDNA ratio on the Y axis. The error bars indicate standard errors. The X axis indicates time (hours).

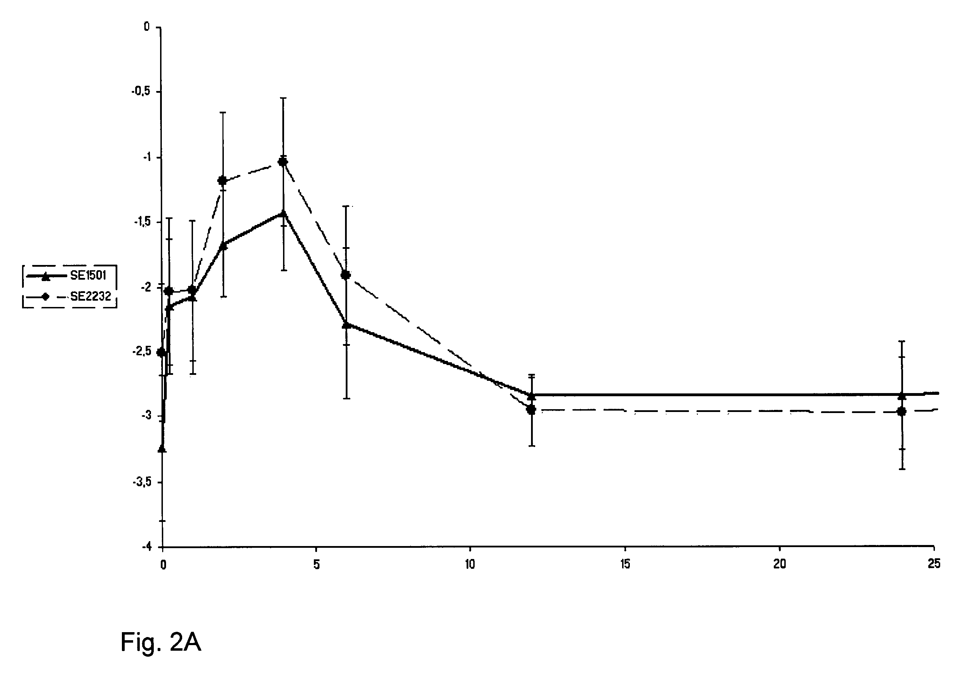

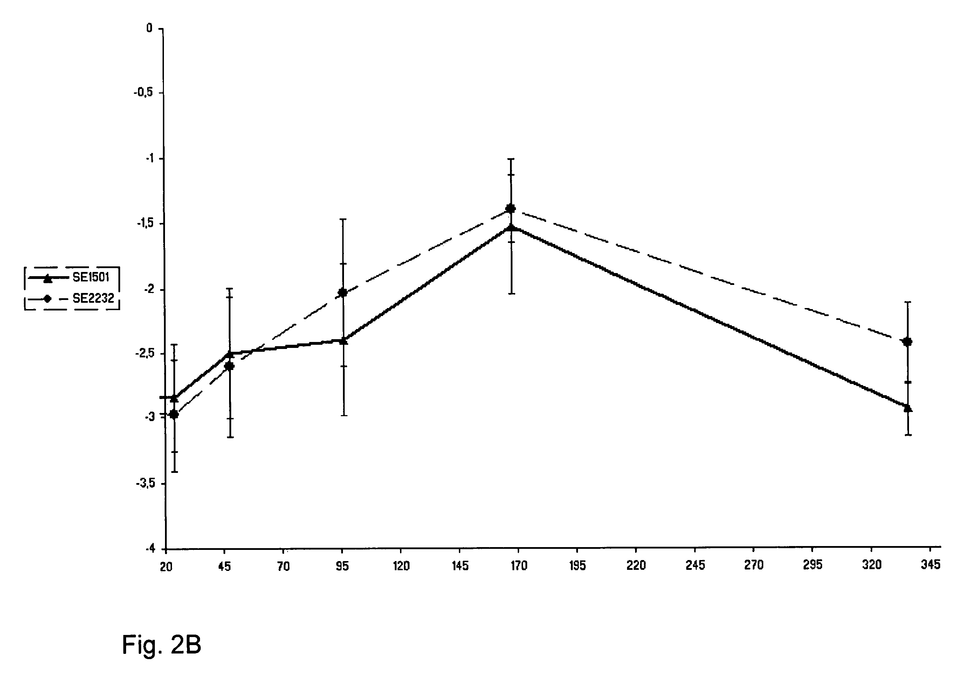

**FIG. 2****.** Evolution of the in vivo expression of genes SE1501 and SE2232 during the first 24 hours after implantation (A) and between 24 hours and 2 weeks (B). The expression level at any time point is represented as the log10 cDNA/gDNA ratio (Y axis) versus time (in minutes (A) and hours (B)) on the X axis. The error bars indicate standard errors.

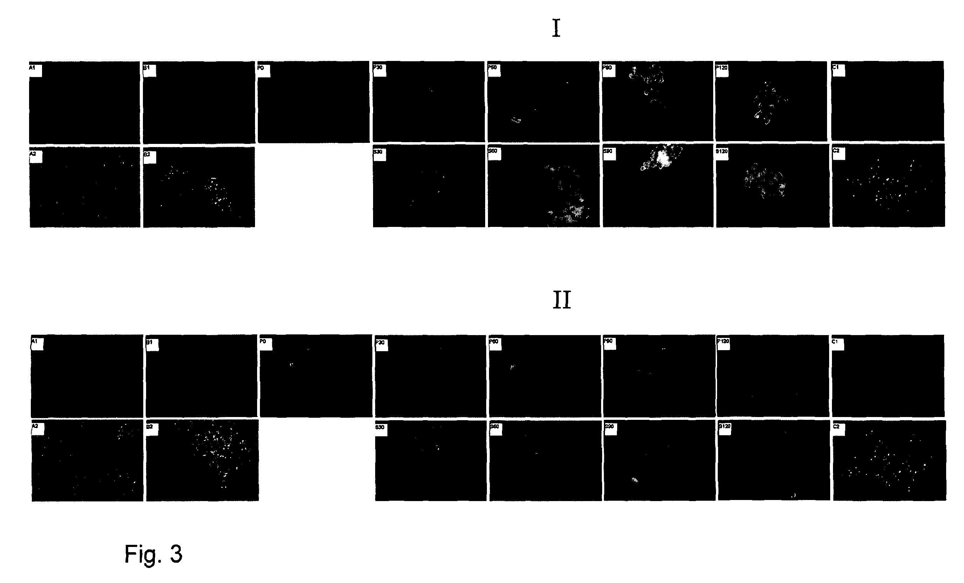

**FIG. 3****.** Images taken by fluorescence microscopy from planktonic (P) and sessile (S) bacteria at different time points for SE1501 (I) and SE2232 (II). Images A1 and A2 shows bacterial cells plus control rabbit antibody and FITC-labelled goat-anti-rabbit antibody visualized by fluorescence (A1) and yellow light (A2). IB1 and IB2 images were taken from control samples containing only bacterial cells plus RPAbSE1501 and IIB1 and IIB2 images of bacterial cells plus RPAbSE2232. Images of C1 and C2 were taken from control sample of cells plus only FITC-labelled goat-anti-rabbit antibody visualized by fluorescence and yellow light.

Cells from sessile samples are in a more condensed and aggregated condition. In the images of planktonic samples for SE1501 we can see that protein expression of SE1501 in the first 30 min of the experiment decreased and then increased and peaked at the end of experiment. In images taken from sessile samples it is clear that the expression of protein SE1501 gradually increased over time. In the images of planktonic samples for SE2232, it can be observed that the expression of SE2232 decreased at the beginning of the experiment and increased in samples taken at 90 and 120 min. In images taken from sessile samples we can see that expression of SE2232 after a decrease at 30 min increased again .

Expressions of both proteins (SE1501 and SE2232) in all sessile samples were higher than in their planktonic counterpart samples.

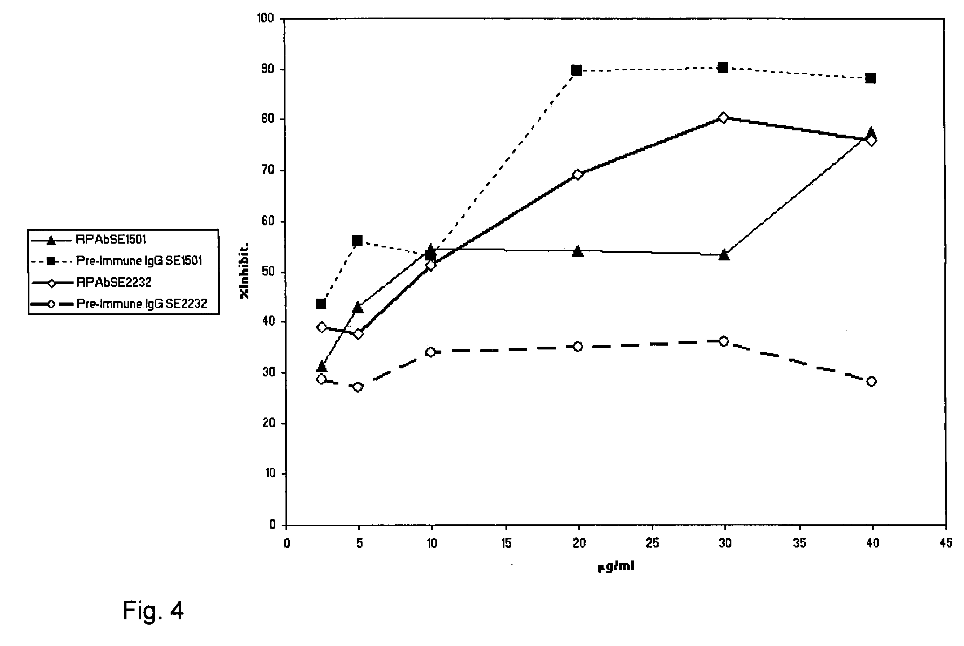

**FIG. 4****.** *S. epidermis* 10b biofilm inhibition by IgG fractions purified from pre and post-immune sera of rabbits immunized against recombinant gene products of SE1501 and SE2232 in a 96-well polystyrene plate. Bacteria were suspended in 0.9% NaCl containing the indicated concentrations of polyclonal antibodies and incubated for 2 h at 4°C and then overnight at 37°C. Formation of the biofilm was measured with safranin O. Percent inhibition of biofilm accumulation was determined from the formula (A₄₉₂, positive - A₄₉₂, antibody)/(A₄₉₂, positive - A₄₉₂, negative) x 100%. (Sun, D.M. et al., 2005, Clin. Diagn. Lab. Immunol. 12:93-100).

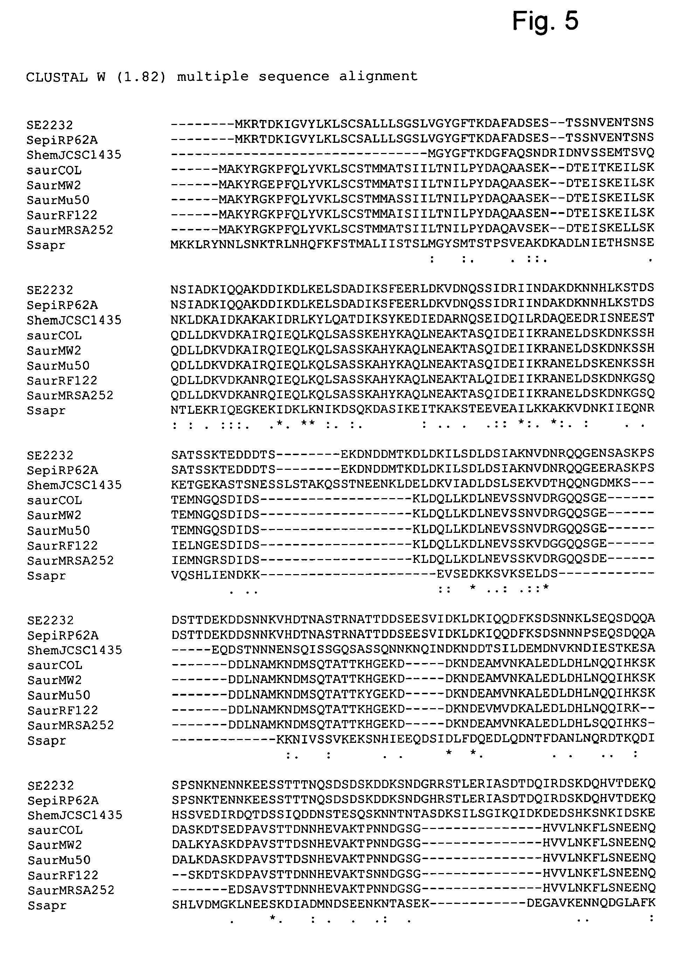

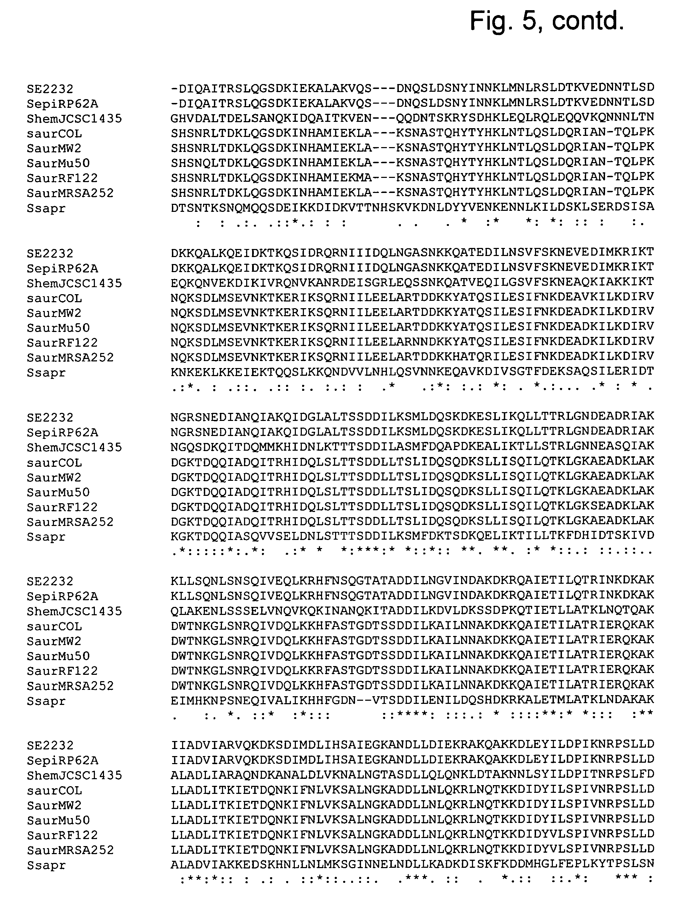

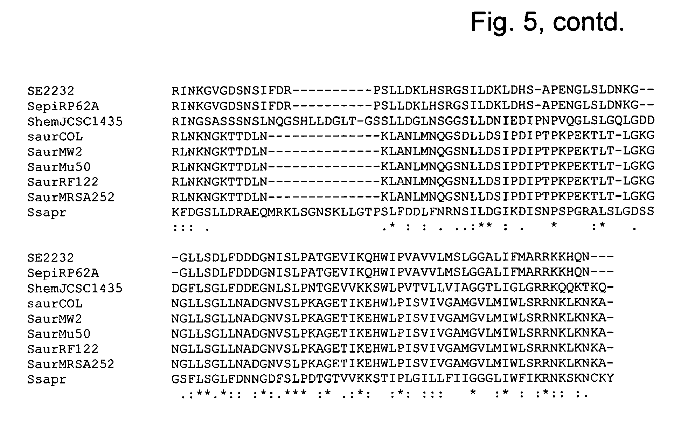

**FIG. 5****.** Sequence alignment (Clustal W, version 1.82) of SE2232 and highly homologous *Staphylococcus* proteins. SepiRP62A is a cell wall surface anchor family protein of *S*. *epidermis* RP62A; ShemJCSC1435 is the hypothetical protein SH0356 of *S. hemolyticus* JCSC1435; SaurCOL is the hypothetical LPxTG protein SA04HSC_02982 from *S. aureus* ssp. *aureus* COL; SaurMW2 is the hypothetical protein MW2567 of *S. aureus* ssp. *aureus* MW2; SaurMu50 is the hypothetical protein SAV2646 from *S. aureus* ssp. *aureus* Mu50; SaurRF122 is a protein surface anchored protein of *S*. *aureus* RF122; SaurMRSA252 is a putative protein of *S*. *aureus* ssp. *aureus* MRSA252; Ssapr is the hypothetical protein SSPP105 from *S. saprophyticus* ssp. *saprophyticus* ATCC 15305.

### DETAILED DESCRIPTION OF THE INVENTION

"LPxTG motif" as used herein refers to the occurrence of a sequence of 5 amino acids in a microbial protein, where said 5 amino acids are: Leu-Pro-Xaa-Thr-Gly, in which Xaa indicates any natural occurring amino acid.

"Identity" or "sequence identity", "similarity" and "sequence similarity", "homology" or "sequence homology" all refer to the degree of correspondence of one stretch of nucleotides or amino acids with another sequence of nucleotides or amino acids. Highly homologous in this sense means that an amino acid sequence has a sequence homology of more than 50%, preferably more than 70%, more preferably more than 80% and most preferably more than 90% with the above mentioned sequence.

"Identity" and "similarity" can be readily calculated by known methods (Computational Molecular Biology, Lesk, A. M., ed., Oxford University Press, New York, 1988; Biocomputing: Informatics and Genome Projects, Smith, D. W., ed., Academic Press, New York, 1993). While there exist a number of methods to measure identity and similarity between two sequences, both terms are well known to skilled artisans. Methods commonly employed to determine identity or similarity between sequences include, but are not limited to those disclosed in Carillo, H., and Lipman, D., SIAM J. Applied Math., 48:1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, GCG program package (Devereux et al., Nucleic Acids Research 12(1): 387, 1984), BLASTP, BLASTN, and FASTA (Atschul et al., J. Molec. Biol. 215: 403-410, 1990). The BLAST X program is publicly available from NCBI and other sources (BLAST Manual, Altschul et al., NCBI NLM NIH Bethesda, Md. 20894; Altschul et al., J. Mol. Biol. 215: 403-410, 1990). BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, 1993 Comput. Chem. 17:149-163) and XNU (Claverie and States, 1993 Comput. Chem. 17:191-201) low-complexity filters can be employed alone or in combination. The BLAST program used in the present invention for determining homology between proteins is BLASTP 2.2.16 (Altschul, S.F. et al. 1997, Nucleic Acids Res. 25:3389-3402; Schäffer, A. A. et al., 2001, Nucleic Acids Res. 29:2994-3005)

As used herein, 'sequence identity' or 'identity' or 'homology' in the context of two protein sequences (or nucleotide sequences) includes reference to the residues in the two sequences which are the same when aligned for maximum correspondence over a specified comparison window. When percentage of sequence identity is used in reference to proteins it is recognised that residue positions which are not identical often differ by conservative amino acid substitutions, where amino acids are substituted for other amino acid residues with similar chemical properties (e.g. charge or hydrophobicity) and therefore do not change the functional properties of the molecule. Where sequences differ in conservative substitutions, the percentage sequence identity may be adjusted upwards to correct for the conservative nature of the substitutions. Sequences, which differ by such conservative substitutions are said to have 'sequence similarity' or 'similarity'. Means for making these adjustments are well known to persons skilled in the art. Typically this involves scoring a conservative substitution as a partial rather than a full mismatch, thereby increasing the percentage sequence identity. Thus, for example, where an identical amino acid is given a score of 1 and a non-conservative substitution is give a score of zero, a conservative substitution is given a score between 0 and 1. The scoring of conservative substitutions is calculated, e.g. according to the algorithm of Meyers and Miller (Computer Applic. Biol. Sci. 4:11-17, 1988).

"CoNS" bacteria or "coagulase-negative" bacteria are bacteria of the genus *Staphylococcus* except for *S*. *aureus* and *S*. *intermedius.* However, all bacteria of the genus *Staphylococcus* are capable of biofilm formation.

By "biofilm" is meant the mass of microorganisms attached to a surface, such as a surface of a medical device, and the associated extracellular substances produced by one or more of the attached microorganisms. The extracellular substances are typically polymeric substances and commonly comprise a matrix of complex polysaccharides, proteinaceous substances and glycopeptides. This matrix or biofilm is also commonly referred to as "glycocalyx." Biofilms can not only occur on insertable medical devices ("foreign bodies"), but also appear as a consequence of a bacterial infection on structures of the human or animal body itself. Clinically important are biofilm formation by *S. aureus* connected with otitis media and vaginal infections.

Biofilm formation on the surfaces of implantable or insertable medical devices ("foreign bodies") adapted for long-term implantation, e.g., from about 30 days to 12 months or longer, can result in eventual encrustation and failure of the device. Further, the proliferation of microbes within the biofilm can lead to localized infections as well as difficult to treat systemic infections. The extracellular substances that comprise the biofilm matrix can act as a barrier that protects and isolates the microorganisms housed in the biofilm from normal immunological defence mechanisms, such as antibodies and phagocytes, as well as from antimicrobial agents including surfactants, biocides and antibiotics. The biofilm also facilitates the growth and proliferation of microbes housed within the biofilm.

The present invention substantially reduces the risk of biofilm accumulation such as on the surfaces of a medical device adapted for long term implantation, and the resultant likelihood of premature failure of the device due to encrustation and occlusion by such biofilm. In some preferred embodiments of the present invention, the medical device is intended to remain implanted for a relatively long period of from about 30 days to about 12 months or longer. However, it is understood that the device may be implanted for a period of 30 days or shorter as well. Especially important in this respect is the application of the invention in neonates, where catheter-mediated CoNS infections occur in the first week after birth.

As indicated above, *Staphylococcus* spp., especially *S*. *epidermis, S. aureus, S. saprophyticus* and *S*. *hemolyticus* plays a major role in biofilm formation on foreign bodies, and essentially the cell wall associated (CWA) proteins play a major role during the early stages of *Staphylococcus* colonization and the later establishment of disease. One of the common factors of the known CWA proteins Aap, Bhp, SdrF and SdrG is that they are LPxTG motif containing proteins, indicating cell wall anchoring. In publicly available genomes of *S*. *epidermidis* strains RP62A and ATCC 12228, eleven and ten genes encoding LPxTG proteins have been identified respectively, of which eight genes, (SE2395 or *sdr*F, SE0331 or *sdr*G, SE0175 or *aap*, SE1429, SE2162, SE2232, SE0828 and SE1628) are present in both strains and five in only one of the two strains. Two hypothetical proteins, SE1500 and SE1501, are present in strain ATCC 12228 but not in RP62A, whereas the ORF's encoding SERP2392 (Bhp), SERP1482 and SERP1654 are only found in the *S*. *epidermidis* RP62A genome. Four genes encode the previously described proteins (Aap, Bhp, SdrF and SdrG), while the remaining ones have not been characterized yet.

We investigated the gene expression in planktonic and sessile bacteria of two hypothetical LPxTG proteins i.c. SE1501 and SE2232. The SE1501 gene encodes a hypothetical protein of 415 amino acids. The protein is similar to a few other known proteins including1,4-alpha-glucan branching enzyme from *Clostridium beijerincki* and 2',3'-cyclic-nucleotide 2'-phosphodiesterase from *Clostridium perfringens* (respectively 48% and 49% identity). The SE2232 gene encodes a hypothetical protein of 676 amino acids (see Fig. 5). This protein exhibits some similarity to the much larger AAS surface protein of *Staphylococcus saprophyticus* (Hell, W. et al., 1998, Mol. Microbiol. 29:871-881) and staphylococcal fibronectin binding proteins such as AtlC from *Staphylococcus caprae* (Allignet, J. et al., 2001, Infect. Immun. 69:712-718) with respectively 43% and 44% identity. Highest homology, however, is found to some putative or hypothetical proteins from *S*. *aureus, S. hemolyticus* and *S*. *saprophyticus,* as is shown in Figure 5. The e-values obtained from a BLASTP comparison of SE2232 with the various proteins listed were 2e⁻⁷⁸ for *S. hemolyticus* JCSC1435, 5e⁻⁷⁷ for *S. aureus* COL, 8e⁻⁷⁷ for *S. aureus* MW2 and *S*. *aureus* Mu50, 3e⁻⁷⁵ for *S*. *aureus* RF122 and MRSA252 and 3e⁻⁵³ for *S. saprophyticus*.

As is shown in the experimental part, it is surprising that inhibiting the SE2232 protein (through rabbit polyclonal antibody serum) was able to significantly prevent biofilm formation, whereas inhibition of SE1501 did not yield significant results. The invention thus provides a method to prevent biofilm formation on a surface by coating said surface with antibodies against SE2232 or antibodies generated against any of the proteins listed in Fig. 5.

As used herein, the terms "antibody" and "antibodies" refer to monoclonal antibodies, multispecific antibodies (*e.g.*, bi-specific), human antibodies, humanized antibodies, camelised antibodies, chimeric antibodies, single-chain Fvs (scFv), single chain antibodies, synthetic antibodies, single domain antibodies, Fab fragments, F(ab) fragments, disulfide-linked Fvs (sdFv), and anti-idiotypic (anti-Id) antibodies, and epitope-binding fragments of any of the above. In particular, antibodies include immunoglobulin molecules and immunologically active fragments of immunoglobulin molecules, i.e., molecules that contain an antigen binding site. Immunoglobulin molecules can be of any type *(e.g.,* IgG, IgE, IgM, IgD, IgA and IgY), class (*e*.*g*., IgG₁, IgG₂, IgG₃, IgG₄, IgA₁ and IgA₂) or subclass.

Antibodies of the invention include, but are not limited to, monoclonal antibodies, multispecific antibodies, synthetic antibodies, human antibodies, humanized antibodies, chimeric antibodies, single-chain Fvs (scFv), single chain antibodies, Fab fragments, F(ab') fragments, disulfide-linked Fvs (sdFv), and anti-idiotypic (anti-Id) antibodies (including, *e*.*g*., anti-Id antibodies to antibodies of the invention), and epitope-binding fragments of any of the above. In particular, antibodies of the present invention include immunoglobulin molecules and immunologically active portions of immunoglobulin molecules, *i*.*e*., molecules that contain an antigen binding site that immunospecifically binds to an SE2232 antigen or an antigen against any of the proteins listed in Fig. 5. The immunoglobulin molecules of the invention can be of any type (*e*.*g*., IgG, IgE, IgM, IgD, IgA and IgY), class (*e*.*g*., IgG₁, IgG₂, IgG₃, IgG₄, IgA₁ and IgA₂) or subclass of immunoglobulin molecule.

The antibodies of the invention may be from any animal origin including birds and mammals (*e.g*., human, murine, donkey, sheep, rabbit, goat, guinea pig, camel, horse, or chicken). As used herein, "human" antibodies include antibodies having the amino acid sequence of a human immunoglobulin and include antibodies isolated from human immunoglobulin libraries (including, but not limited to, synthetic libraries of immunoglobulin sequences homologous to human immunoglobulin sequences) or from mice that express antibodies from human genes.

In certain embodiments, high potency antibodies can be used in the methods of the invention. For example, high potency antibodies can be produced by genetically engineering appropriate antibody gene sequences and expressing the antibody sequences in a suitable host. The antibodies produced can be screened to identify antibodies with, *e*.*g*., high kₒₙ values in a BIAcore assay.
In certain embodiments, an antibody to be used with the methods of the present invention or fragment thereof has an affinity constant or Kₐ (kₒₙ/k_{off}) of at least 10² M⁻¹, at least 5 X 10² M⁻¹, at least 10³ M⁻¹, at least 5 X 10³ M⁻¹, at least 10⁴ M⁻¹, at least 5 X 10⁴ M⁻¹, at least 10⁵ M⁻¹, at least 5 X 10⁵ M⁻¹, at least 10⁶ M⁻¹, at least 5 X 10⁶ M⁻¹, at least 10⁷ M⁻¹, at least 5 X 10⁷ M⁻¹, at least 10⁸ M⁻¹, at least 5 X 10⁸ M⁻¹, at least 10⁹ M⁻¹, at least 5 X 10⁹ M⁻¹, at least 10¹⁰ M⁻¹, at least 5 X 10¹⁰ M⁻¹, at least 10¹¹ M⁻¹, at least 5 X 10¹¹ M⁻¹, at least 10¹² M⁻¹, at least 5 X 10¹² M⁻¹, at least 10¹³ M⁻¹, at least 5 X 10¹³ M⁻¹, at least 10¹⁴ M⁻¹, at least 5 X 10¹⁴ M⁻¹, at least 10¹⁵ M⁻¹, or at least 5 X 10¹⁵ M⁻¹. In yet another embodiment, an antibody to be used with the methods of the invention or fragment thereof has a dissociation constant or K_{d} (k_{off}/kₒₙ) of less than 10⁻² M, less than 5 X 10⁻² M, less than 10⁻³ M, less than 5 X 10⁻³ M, less than 10⁻⁴ M, less than 5 X 10⁻⁴ M, less than 10⁻⁵ M, less than 5 X 10⁻⁵ M, less than 10⁻⁶ M, less than 5 X 10⁻⁶ M, less than 10⁻⁷ M, less than 5 X 10⁻⁷ M, less than 10⁻⁸ M, less than 5 X 10⁻⁸ M, less than 10⁻⁹ M, less than 5 X 10⁻⁹ M, less than 10⁻¹⁰ M, less than 5 X 10⁻¹⁰ M, less than 10⁻¹¹ M, less than 5 X 10⁻¹¹ M, less than 10⁻¹² M, less than 5 X 10⁻¹² M, less than 10⁻¹³ M, less than 5 X 10⁻¹³ M, less than 10⁻¹⁴ M, less than 5 X 10⁻¹⁴ M, less than 10⁻¹⁵ M, or less than 5 X 10⁻¹⁵ M.
In certain embodiments, an antibody to be used with the methods of the invention or fragment thereof that has a median effective concentration (EC₅₀) of less than 0.01 nM, less than 0.025 nM, less than 0.05 nM, less than 0.1 nM, less than 0.25 nM, less than 0.5 nM, less than 0.75 nM, less than 1 nM, less than 1.25 nM, less than 1.5 nM, less than 1.75 nM, or less than 2 nM, in an *in vitro* biofilm inhibition assay (see experimental part). The median effective concentration is the concentration of antibody or antibody fragments that inhibits 50% of the biofilm formation in said biofilm inhibition assay. In a preferred embodiment, an antibody to be used with the methods of the invention or fragment thereof has an EC₅₀ of less than 0.01 nM, less than 0.025 nM, less than 0.05 nM, less than 0.1 nM, less than 0.25 nM, less than 0.5 nM, less than 0.75 nM, less than 1 nM, less than 1.25 nM, less than 1.5 nM, less than 1.75 nM, or less than 2 nM, in biofilm inhibition assay.

The antibodies to be used with the methods of the invention include derivatives that are modified, i.e, by the covalent attachment of any type of molecule to the antibody such that covalent attachment. For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, synthesis in the presence of tunicamycin, etc. Additionally, the derivative may contain one or more non-classical amino acids.

The present invention also provides antibodies of the invention or fragments thereof that comprise a framework region known to those of skill in the art. In certain embodiments, one or more framework regions, preferably, all of the framework regions, of an antibody to be used in the methods of the invention or fragment thereof are human. In certain other embodiments of the invention, the fragment region of an antibody of the invention or fragment thereof is humanized. In certain embodiments, the antibody to be used with the methods of the invention is a synthetic antibody, a monoclonal antibody, an intrabody, a chimeric antibody, a human antibody, a humanized chimeric antibody, a humanized antibody, a glycosylated antibody, a multispecific antibody, a human antibody, a single-chain antibody, or a bispecific antibody. In certain embodiments of the invention, the antibodies to be used with the invention have half-lives in a mammal, preferably a human, of greater than 12 hours, greater than 1 day, greater than 3 days, greater than 6 days, greater than 10 days, greater than 15 days, greater than 20 days, greater than 25 days, greater than 30 days, greater than 35 days, greater than 40 days, greater than 45 days, greater than 2 months, greater than 3 months, greater than 4 months, or greater than 5 months. Antibodies or antigen-binding fragments thereof having increased *in vivo* half-lives can be generated by techniques known to those of skill in the art. For example, antibodies or antigen-binding fragments thereof with increased *in vivo* half-lives can be generated by modifying (*e*.*g*., substituting, deleting or adding) amino acid residues identified as involved in the interaction between the Fc domain and the FcRn receptor (see, e.g., PCT Publication No. WO 97/34631 and U.S. Patent Application No.: 10/020,354, entitled "Molecules with Extended Half-Lives, Compositions and Uses Thereof", filed December 12, 2001, by Johnson et al., which are incorporated herein by reference in their entireties). Such antibodies or antigen-binding fragments thereof can be tested for binding activity to SE2232 antigens or any of the other proteins listed in Fig. 5, as well as for *in vivo* efficacy using methods known to those skilled in the art, for example, by assays as described herein.
Further, antibodies or antigen-binding fragments thereof with increased *in vivo* half-lives can be generated by attaching to said antibodies or antibody fragments polymer molecules such as high molecular weight polyethyleneglycol (PEG). PEG can be attached to said antibodies or antibody fragments with or without a multifunctional linker either through site-specific conjugation of the PEG to the N- or C-terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, *e*.*g*., size exclusion or ion-exchange chromatography. PEG-derivatizated antibodies or antigen-binding fragments thereof can be tested for binding activity to SE2232 antigens as well as for *in vivo* efficacy using methods known to those skilled in the art, for example, by assays as described herein.

In certain embodiments, the antibodies to be used with the methods of the invention are fusion proteins comprising an antibody or fragment thereof that immunospecifically binds to a SE2232 antigen and a heterologous polypeptide. Preferably, the heterologous polypeptide that the antibody or antibody fragment is fused to is useful for coating of foreign bodies or devices inserted into the human or mammalian body.

In certain embodiments, antibodies to be used with the methods of the invention are single-chain antibodies(sFv). The design and construction of a single-chain antibody is described in Marasco et al, 1993, Proc Natl Acad Sci 90:7889-7893, which is incorporated herein by reference in its entirety. The sFv typically comprises a single peptide with the sequence V_{H} -linker-V_{L} or V_{L}-linker-V_{H}. The linker is chosen to permit the heavy chain and light chain to bind together in their proper conformational orientation (see for example, Huston, et al., 1991, Methods in Enzym. 203:46-121, which is incorporated herein by reference). In a further embodiment, the linker can span the distance between its points of fusion to each of the variable domains (*e*.*g*., 3.5 nm) to minimize distortion of the native Fv conformation. In such an embodiment, the linker is a polypeptide of at least 5 amino acid residues, at least 10 amino acid residues, at least 15 amino acid residues, or greater. In a further embodiment, the linker should not cause a steric interference with the V_{H} and V_{L} domains of the combining site. In such an embodiment, the linker is 35 amino acids or less, 30 amino acids or less, or 25 amino acids or less. Thus, in a most preferred embodiment, the linker is between 15-25 amino acid residues in length. In a further embodiment, the linker is hydrophilic and sufficiently flexible such that the V_{H} and V_{L} domains can adopt the conformation necessary to detect antigen. Examples of linkers include, but are not limited to, those sequences disclosed in Table 1.

**Table 1**

| **Sequence** |
|---|
| (Gly Gly Gly Gly Ser)₃ |
| Glu Ser Gly Arg Ser Gly Gly Gly Gly Ser Gly Gly Gly Gly Ser |
| Glu Gly Lys Ser Ser Gly Ser Gly Ser Glu Ser Lys Ser Thr |
| Glu Gly Lys Ser Ser Gly Ser Gly Ser Glu Ser Lys Ser Thr Gln |
| Glu Gly Lys Ser Ser Gly Ser Gly Ser Glu Ser Lys Val Asp |
| Gly Ser Thr Ser Gly Ser Gly Lys Ser Ser Glu Gly Lys Gly |
| Lys Glu Ser Gly Ser Val Ser Ser Glu Gln Leu Ala Gln Phe Arg Ser Leu Asp |
| Glu Ser Gly Ser Val Ser Ser Glu Glu Leu Ala Phe Arg Ser Leu Asp |

The antibodies to be used with the methods of the invention or fragments thereof can be produced by any method known in the art for the synthesis of antibodies, in particular, by chemical synthesis or preferably, by recombinant expression techniques.

Polyclonal antibodies to an SE2232 antigen or an antigen from the proteins listed in Fig. 5 can be produced by various procedures well known in the art. For example, an SE2232 antigen can be administered to various host animals including, but not limited to, rabbits, mice, rats, etc. to induce the production of sera containing polyclonal antibodies specific for the SE2232 antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, vitamin **E** and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Methods for producing and screening for specific antibodies using hybridoma technology are routine and well known in the art. Briefly, mice can be immunized with an SE2232 antigen and once an immune response is detected, *e.g*., antibodies specific for the SE2232 antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

In certain embodiments, a method of generating monoclonal antibodies comprises culturing a hybridoma cell secreting an antibody of the invention wherein, preferably, the hybridoma is generated by fusing splenocytes isolated from a mouse immunized with an SE2232 antigen with myeloma cells and then screening the hybridomas resulting from the fusion for hybridoma clones that secrete an antibody able to bind an SE2232 antigen.

Antibody fragments which recognize specific SE2232 epitopes or epitopes of any of the proteins listed in Fig. 5, may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies to be used with the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (*e*.*g*., human or murine cDNA libraries of lymphoid tissues). The DNA encoding the VH and VL domains are recombined together with an scFv linker by PCR and cloned into a phagemid vector (*e*.*g*., p CANTAB 6 or pComb 3 HSS). The vector is electroporated in *E. coli* and the *E*. *coli* is infected with helper phage. Phage used in these methods are typically filamentous phage including fd and M13 and the VH and VL domains are usually recombinantly fused to either the phage gene III or gene VIII. Phage expressing an antigen binding domain that binds to a SE2232 antigen can be selected or identified with antigen, e.g., using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; PCT application No. PCT/GB91/O1 134; PCT publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described below. Techniques to recombinantly produce Fab, Fab' and F(ab')2 fragments can also be employed using methods known in the art such as those disclosed in PCT publication No. WO 92/22324; Mullinax et al., 1992, BioTechniques 12(6):864-869; Sawai et al., 1995, AJRI 34:26-34; and Better et al., 1988, Science 240:1041-1043 (said references incorporated by reference in their entireties). To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e*.*g*., the human gamma 4 constant region, and the PCR amplified VL domains can be cloned into vectors expressing a VL constant region, e.g., human kappa or lamba constant regions. Preferably, the vectors for expressing the VH or VL domains comprise an EF-1α promoter, a secretion signal, a cloning site for the variable domain, constant domains, and a selection marker such as neomycin. The VH and VL domains may also cloned into one vector expressing the necessary constant regions. The heavy chain conversion vectors and light chain conversion vectors are then co-transfected into cell lines to generate stable or transient cell lines that express full-length antibodies, e.g., IgG, using techniques known to those of skill in the art.

For some uses, including *in vivo* use of antibodies in humans and *in vitro* detection assays, it may be preferable to use human or chimeric antibodies. Completely human antibodies are particularly desirable for coating foreign bodies which are introduced into human subjects. Human antibodies can be made by a variety of methods known in the art including phage display methods described above using antibody libraries derived from human immunoglobulin sequences or synthetic sequences homologous to human immunoglobulin sequences. See also U.S. Patent Nos. 4,444,887 and 4,716,111; and PCT publications WO 98/46645, WO 98/50433, WO 98/24893, WO98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then be bred to produce homozygous offspring which express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see,* e.g., PCT publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, which are incorporated by reference herein in their entireties. In addition, companies such as Medarex, Inc. (Princeton, NJ), Abgenix, Inc. (Freemont, CA) and Genpharm (San Jose, CA) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human (*e*.*g*., murine) antibody and a human immunoglobulin constant region. Methods for producing chimeric antibodies are known in the art. See *e*.*g*., Morrison, 1985, Science 229:1202; Oi et al., 1986, BioTechniques 4:214; Gillies et al., 1989, J. Immunol. Methods 125:191-202; and U.S. Patent Nos. 5,807,715, 4,816,567, and 4,816,397, which are incorporated herein by reference in their entireties. Chimeric antibodies comprising one or more CDRs from human species and framework regions from a non-human immunoglobulin molecule can be produced using a variety of techniques known in the art including, for example, CDR-grafting (EP 239,400; PCT publication No. WO 91/09967; and U.S. Patent Nos. 5,225,539, 5,530,101, and 5,585,089), veneering or resurfacing (EP 592,106; EP 519,596; Padlan, 1991, Molecular Immunology 28(4/5):489-498; Studnicka et al., 1994, Protein Engineering 7(6):805-814; and Roguska et al., 1994, PNAS 91:969-973), and chain shuffling (U.S. Patent No. 5,565,332). Often, framework residues in the framework regions will be substituted with the corresponding residue from the CDR donor antibody to alter, preferably improve, antigen binding. These framework substitutions are identified by methods well known in the art, e.g., by modelling of the interactions of the CDR and framework residues to identify framework residues important for antigen binding and sequence comparison to identify unusual framework residues at particular positions. (See, e.g., Queen et al., U.S. Patent No. 5,585,089; and Riechmann et al., 1988, Nature 332:323, which are incorporated herein by reference in their entireties.)
In certain embodiments, a fragment of a protein of SE2232 is used as an immunogen for the generation of antibodies to be used with the methods of the invention. A fragment of a protein of SE2232 to be used as an immunogen can be at least 10, 20, 30, 40, 50, 75, 100, 250, 500, 750, or at least 1000 amino acids in length. In certain embodiments a synthetic peptide of a protein of SE2232 is used as an immunogen.

Polynucleotides encoding antibodies to be used with the invention may be obtained, and the nucleotide sequence of the polynucleotides determined, by any method known in the art. Such a polynucleotide encoding the antibody may be assembled from chemically synthesized oligonucleotides (*e*.*g*., as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR. Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (*e*.*g*., an antibody cDNA library, or a cDNA library generated from, or nucleic acid, preferably poly A+ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, *e*.*g*., a cDNA clone from a cDNA library that encodes the antibody. Amplified nucleic acids generated by PCR may then be cloned into replicable cloning vectors using any method well known in the art.

Once the nucleotide sequence coding for the antibody is determined, said nucleotide sequence may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, a nucleotide coding for one or more of the CDRs is inserted within framework regions using routine recombinant DNA techniques. The framework regions may be naturally occurring or consensus framework regions, and preferably human framework regions (see, *e*.*g*., Chothia et al., 1998, J. Mol. Biol. 278: 457-479 for a listing of human framework regions). Preferably, the polynucleotide generated by the combination of the framework regions and CDRs encodes an antibody that specifically binds to an SE2232 antigen and/or an antigen of one of the proteins listed in Fig. 5. In certain embodiments, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

Recombinant expression of an antibody to be used with the methods of the invention, derivative or analog thereof, (*e.g.*, a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof (preferably, but not necessarily, containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy, the entire light chain, or both the entire heavy and light chains. The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.
A variety of host-expression vector systems may be utilized to express the antibody molecules of the invention (see, *e*.*g*., U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria (*e*.*g*., *E. coli* and *B. subtilis)* transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors (*e*.*g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e*.*g*., COS, CHO, BHK, 293, NS0, and 3T3 cells) harboring recombinant expression constructs containing promoters derived from the genome of mammalian cells (*e.g.*, metallothionein promoter) or from mammalian viruses (*e*.*g*., the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2). In a specific embodiment, the expression of nucleotide sequences encoding antibodies or antigen-binding fragments thereof which immunospecifically bind to one or more SE2232 antigens and/or antigens of the proteins listed in Fig. 5 is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of antibody molecules for coating of devices, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e*.*g*., region El or E3) will result in a recombinant virus that is viable and capable of expressing the antibody molecule in infected hosts (*e*.*g*., see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, e.g., Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g.*, glycosylation) and processing (*e.g.*, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0 (a murine myeloma cell line that does not endogenously produce any immunoglobulin chains), CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e*.*g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), hypoxanthineguanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, hgprt- or aprt- cells, respectively. Also, antimetabolite resistance can be used as the basis of selection for the following genes: *dhfr,* which confers resistance to methotrexate (Wigler et al., 1980, Natl. Acad. Sci. USA 77:357; O'Hare et al., 1981, Proc. Natl. Acad. Sci. USA 78:1527); *gpt*, which confers resistance to mycophenolic acid (Mulligan & Berg, 1981, Proc. Natl. Acad. Sci. USA 78:2072); neo, which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

Once an antibody molecule to be used with the methods of the invention has been produced by recombinant expression, it may be purified by any method known in the art for purification of an immunoglobulin molecule, for example, by chromatography (*e.g.*, ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

In certain embodiments, the antibodies to be used with the methods of the invention or fragments thereof are recombinantly fused or chemically conjugated (including both covalently and non-covalently conjugations) to a heterologous polypeptide (or portion thereof, preferably at least 10, at least 20, at least 30, at least 40, at least 50, at least 60, at least 70, at least 80, at least 90 or at least 100 amino acids of the polypeptide) to generate fusion proteins. The fusion does not necessarily need to be direct, but may occur through linker sequences. In certain embodiments, the anti-SE2232-antigen antibody is an antibody conjugate.

Additional fusion proteins of the antibodies to be used with the methods of the invention or fragments thereof may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (*e.g.*, antibodies or antigen-binding fragments thereof with higher affinities and lower dissociation rates). See, generally, U.S. Patent Nos. 5,605,793; 5,811,238; 5,830,721; 5,834,252; and 5,837,458, and Patten et al., Curr. Opinion Biotechnol. 8:724-33 (1997); Harayama, Trends Biotechnol. 16(2):76-82 (1998); Hansson, et al., J. Mol. Biol. 287:265-76 (1999); and Lorenzo and Blasco, Biotechniques 24(2):308- 13 (1998) (each of these patents and publications are hereby incorporated by reference in its entirety). In one embodiment, antibodies or antigen-binding fragments thereof, or the encoded antibodies or antigen-binding fragments thereof, may be altered by being subjected to random mutagenesis by error-prone PCR, random nucleotide insertion or other methods prior to recombination. In another embodiment, one or more portions of a polynucleotide encoding an antibody or antibody fragment, which portions immunospecifically bind to an SE2232 antigen and/or an antigen of one or more of the proteins listed in Fig. 5 may be recombined with one or more components, motifs, sections, parts, domains, fragments, etc. of one or more heterologous molecules.

Moreover, the antibodies to be used with the methods of the present invention or fragments thereof can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemaggiutinin"HA" tag, which corresponds to an epitope derived from the influenza hemagglutinin protein (Wilson et al., 1984, Cell 37:767) and the "flag" tag.

An antibody or fragment thereof may be conjugated to a therapeutic moiety such as, but not limited to, a antibacterial compound or a therapeutic agent. Antibiotics to be used comprise bacteriocins, such as nisin and pediocin; antibiotics, such as penicillin, erythromycin, ampicillin, isoniazid, tetracycline, sulphonamides and chloramphenicol; vegetable toxins, such as defensins, lectins, and anti-fungal proteins; H₂O₂-producing enzymes such as oxidases; sodium diacetate; sodium nitrite; lysozyms and antimicrobial substances from spices. Further, an antibody to be used with the methods of the invention or fragment thereof may be conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, but are not limited to, a protein such as tumor necrosis factor, α-interferon, β-interferon, nerve growth factor, platelet derived growth factor, tissue plasminogen activator, an apoptotic agent, *e*.*g*., TNF-α, TNF-ß, AIM I (see, International Publication No. WO 97/33899), AIM II (see, International Publication No. WO 97/34911), Fas Ligand (Takahashi et al., 1994, J. Iminunol., 6:1567-1574), and VEGI (see, International Publication No. WO 99/23105), a thrombotic agent or an anti-angiogenic agent, *e*.*g*., angiostatin or endostatin; or, a biological response modifier such as, for example, a lymphokine (*e*.*g*., interleukin-1 ("IL- 1"), interleukin-2 ("IL-2"), interleukin-6 ("IL-6"), granulocyte macrophage colony stimulating factor ("GM-CSF"), and granulocyte colony stimulating factor ("G-CSF")), or a growth factor (*e*.*g*., growth hormone ("GH")).

Techniques for conjugating such therapeutic moieties to antibodies are well known, see, *e.g*., Hellstrom et al., "Antibodies For Drug Delivery", in Controlled Drug Delivery (2nd Ed.), Robinson et al. (eds.), pp. 623-53 (Marcel Dekker, Inc. 1987) and Thorpe et al., 1982, Immunol. Rev. 62:119-58.

One embodiment of the invention provides in medical devices or foreign bodies that are coated with antibodies, as discussed above, that immunospecifically bind to SE2232 and/or one or more of the proteins listed in Fig. 5 or homologues thereof. For this invention homologues of the proteins listed in Fig. 5 are defined as proteins which have an e-value of 1e⁻⁵⁰ or lower, preferably 1e⁻⁷⁵ or lower, in a BLASTP comparison with one or more of the proteins listed in Fig. 5.

Medical devices or polymeric biomaterials to be coated with the antibodies described herein include, but are not limited to, staples, sutures, replacement heart valves, cardiac assist devices, hard and soft contact lenses, intraocular lens implants (anterior chamber or posterior chamber), other implants such as corneal inlays, kerato-prostheses, vascular stents, epikeratophalia devices, glaucoma shunts, retinal staples, scleral buckles, dental prostheses, thyroplastic devices, laryngoplastic devices, vascular grafts, cerebrovascular shunts, soft and hard tissue prostheses including, but not limited to, pumps, electrical devices including stimulators and recorders, auditory prostheses, pacemakers, artificial larynx, dental implants, mammary implants, penile implants, cranio/facial tendons, artificial joints, tendons, ligaments, menisci, and disks, artificial bones, artificial organs including artificial pancreas, artificial hearts, artificial limbs, and heart valves; stents, wires, guide wires, intravenous and central venous catheters, laser and balloon angioplasty devices, vascular and heart devices (tubes, catheters, balloons), ventricular assists, blood dialysis components, blood oxygenators, urethral/ureteral/urinary devices (Foley catheters, stents, tubes and balloons), airway catheters (endotracheal and tracheostomy tubes and cuffs), enteral feeding tubes (including nasogastric, intragastric and jejunal tubes), wound drainage tubes, tubes used to drain the body cavities such as the pleural, peritoneal, cranial, and pericardial cavities, blood bags, test tubes, blood collection tubes, vacutainers, syringes, needles, pipettes, pipette tips, and blood tubing. The medical devices may be formed of any suitable metallic materials or non-metallic materials known to persons skilled in the art. Examples of metallic materials include, but are not limited to, tivanium, titanium, and stainless steel, and derivatives or combinations thereof. Examples of non-metallic materials include, but are not limited to, thermoplastic or polymeric materials such as rubber, plastic, polyesters, polyethylene, polyurethane, silicone, Gortex (polytetrafluoroethylene), Dacron^{™}, (polyethylene tetraphthalate), Teflon (polytetrafluoroethylene), latex, elastomers and Dacron^{™} sealed with gelatin, collagen or albumin, and derivatives or combinations thereof.

Stents include biliary, urethral, ureteral, tracheal, coronary, gastrointestinal and oesophageal stents. The stents may be of any shape or configuration. The stents may comprise a hollow tubular structure which is particularly useful in providing flow or drainage through lumens. Stents may also be coiled or patterned as a braided or woven open network of fibres or filaments or, for example, as an interconnecting open network of articulable segments. Such stent designs may be more particularly suitable for maintaining the patency of a body lumen such as a coronary artery. Thus, stents adapted primarily to provide drainage, in contrast to stents adapted primarily to support a body lumen, will preferably have a continuous wall structure in contrast to an open network wall structure. The stent can be made of any material useful for such purpose including metallic and nonmetallic materials as well as shape memory materials. Useful metallic materials include, but are not limited to, shape memory alloys such as Nitinol^{™}, and other metallic materials including, but not limited to, stainless steel, tantalum, nickel-chrome, or cobalt-chromium, i.e., Elgiloy^{™}. The stent can be made from a single strand or multiple strands of any such material and may be self-expanding.
Stent covers are also a preferred medical device of the present invention. For example, a stent cover may comprise a thin wall tubular or sheath-like structure adapted to be placed over a stent comprising an open mesh stent of knitted, woven or braided design.

The coated devices can be used in both humans and mammals such as pet animals, livestock animals, zoo animals and the like. Preferred non-human mammals are cat, dog, swine and cattle.

It will be understood by those skilled in the art that the term "coated" or "coating", as used herein, means to apply the antibody or binding fragment of the invention to a surface of the device, preferably an outer surface that would be exposed to coagulase-negative staphylococcal infection. The surface of the device does not need to be entirely covered by the antibody or fragment. Coating of antibodies onto substrates is a technique commonly applied in the field of diagnostic assays and biosensors. Any coating method used in these fields may be applied according to the present invention. Preferably, and most simple, coating is provided by immersion of the surface to be coated in a solution comprising the antibody, or, alternatively, by spreading a solution comprising the antibodies onto the surface to be coated.

Natural biofilm formation (i.e. formation of a biofilm which is not deposited onto a "foreign body") occurs sometimes during infection with micro-organisms, especially *S*. *aureus,* especially in otitis media (middle ear infection), vaginal infections and mastitis. In order to pevent or treat such natural biofilm formation, also the antibodies of the invention can be used, especially the antibodies developed against the *S*. *aureus* proteins listed in Fig. 5. However, as alternative to the administration of an antibody to the patient, it is also possible to vaccinate the patient with a subunit vaccine, comprising the protein or an immunogenically active (i.e. antigenic) part thereof.

The route of administration for any one of the embodiments of the vaccine of the present invention includes, but is not limited to, oronasal, intramuscular, intraperitoneal, intradermal, subcutaneous, intravenous, intraarterial, intraocular, intravaginal and oral as well as transdermal or by inhalation or suppository. The preferred routes of administration include oronasal, intramuscular, intraperitoneal, intradermal, intravaginal and subcutaneous injection. The vaccine can be administered by any means that includes, but is not limited to, syringes, nebulizers, misters, needleless injection devices, or microprojectile bombardment gene guns (Biolistic bombardment).

The vaccine for any one of the embodiments of the present invention is formulated in a pharmaceutically accepted carrier according to the mode of administration to be used. One skilled in the art can readily formulate a vaccine that comprises a a protein listed in Fig. 5, a homologous protein thereof, or an immunogenic fragment thereof. In cases where intramuscular injection is preferred, an isotonic formulation is preferred. Generally, additives for isotonicity can include sodium chloride, dextrose, mannitol, sorbitol, and lactose. In particular cases, isotonic solutions such as phosphate buffered saline are preferred. The formulations can further provide stabilizers such as gelatin and albumin. In some embodiments, a vaso-constrictive agent is added to the formulation. The pharmaceutical preparations according to the present invention are provided sterile and pyrogen-free. However, it is well known by those skilled in the art that the preferred formulations for the pharmaceutically accepted carrier which comprise the vaccines of the present invention are those pharmaceutical carriers approved in the regulations promulgated by the United States Department of Agriculture, or equivalent government agency in a foreign country such as Canada or Mexico or any one of the European nations. Therefore, the pharmaceutically accepted carrier for commercial production of the vaccine of the present invention is a carrier that is already approved or will be approved by the appropriate government agency. The vaccine can further be mixed with an adjuvant that is pharmaceutically acceptable. In certain formulations of the vaccine of the present invention, the vaccine is combined with other vaccines to produce a polyvalent vaccine product that can protect subjects not only with respect to biofilm formation, but which will also give protection aginst the micro-organisms, especially *S*. *aureus,* which cause the infection.

Administration is preferably by a single vaccination that produces a full, broad immunogenic response. In another embodiment of the present invention, the subject is treated with a series of vaccinations to produce a full, broad immune response. When the vaccinations are provided in a series, the vaccinations can be provided between about one day to four weeks or longer apart.

The vaccine compositions optionally may include vaccine-compatible pharmaceutically acceptable (i.e., sterile and non-toxic) liquid, semisolid, or solid diluents that serve as pharmaceutical vehicles, excipients, or media. Diluents can include water, saline, dextrose, ethanol, glycerol, and the like. Isotonic agents can include sodium chloride, dextrose, mannitol, sorbitol, and lactose, among others. Stabilizers include albumin, among others. Any adjuvant known in the art may be used in the vaccine composition, including oil-based adjuvants such as Freund's Complete Adjuvant and Freund's Incomplete Adjuvant, mycolate-based adjuvants (*e.g*., trehalose dimycolate), bacterial lipopolysaccharide (LPS), peptidoglycans (i.e., mureins, mucopeptides, or glycoproteins such as N-Opaca, muramyl dipeptide [MDP], or MDP analogs), proteoglycans (*e.g*., extracted from Klebsiella pneumoniae), streptococcal preparations (*e.g*., OK432), Biostim(TM) (*e.g*., 01K2), the "Iscoms" of EP 109 942, EP 180 564 and EP 231 039, aluminum hydroxide, saponin, DEAE-dextran, neutral oils (such as miglyol), vegetable oils (such as arachis oil), liposomes, Pluronic(R) polyols. Adjuvants include, but are not limited to, the RIBI adjuvant system (Ribi Inc.), alum, aluminum hydroxide gel, cholesterol, oil-in water emulsions, water-in-oil emulsions such as, e.g., Freund's complete and incomplete adjuvants, Block co-polymer (CytRx, Atlanta Ga.), SAF-M (Chiron, Emeryville Calif.), AMPHIGEN(R) adjuvant, saponin, Quil A, QS-21 (Cambridge Biotech Inc., Cambridge Mass.), GPI-0100 (Galenica Pharmaceuticals, Inc., Birmingham, Ala.) or other saponin fractions, monophosphoryl lipid A, Avridine lipid-amine adjuvant, heat-labile enterotoxin from E. coli (recombinant or otherwise), cholera toxin, or muramyl dipeptide, among many others. The immunogenic compositions can further include one or more other immunomodulatory agents such as, e.g., interleukins, interferons, or other cytokines. The immunogenic compositions can also include gentamicin and merthiolate. While the amounts and concentrations of adjuvants and additives useful in the context of the present invention can readily be determined by the skilled artisan, the present invention contemplates compositions comprising from about 50 µg to about 2000 µg of adjuvant and preferably about 500 µg/2 ml dose of the vaccine composition. In another preferred embodiment, the present invention contemplates vaccine compositions comprising from about 1 µg/ml to about 60 µg/ml of antibiotic, and more preferably less than about 30 µg/ml of antibiotic.

The immunogenic compositions of the present invention can be made in various forms depending upon the route of administration. For example, the immunogenic compositions can be made in the form of sterile aqueous solutions or dispersions suitable for injectable use, or made in lyophilized forms using freeze-drying techniques. Lyophilized immunogenic compositions are typically maintained at about 4°C., and can be reconstituted in a stabilizing solution, e.g., saline or/and HEPES, with or without adjuvant.

In addition, the immunogenic and vaccine compositions of the present invention can include one or more pharmaceutically-acceptable carriers. As used herein, "a pharmaceutically-acceptable carrier" includes any and all solvents, dispersion media, coatings, adjuvants, stabilizing agents, diluents, preservatives, antibacterial and antifungal agents, isotonic agents, adsorption delaying agents, and the like. The carrier(s) must be "acceptable" in the sense of being compatible with the components of the invention and not deleterious to the subject to be immunized. Typically, the carriers will be will be sterile and pyrogen-free.

The vaccines of the present invention, which are amongs other thought to inhibuit biofilm formation, can be co-administered with any antibiotic treatment. It is envisaged that by preventing the formation of a biofilm, the bacteria are more accessible and thus more prone to antibiotic treatment.

### EXAMPLES

### Example 1: Expression of SE2232 and SE1501 in vitro and in vivo

### Bacteria used.

For all experiments, a previously well-characterized biofilm-producing *S. epidermidis* strain, strain 10b, was used. *S. epidermidis* 10b was isolated from a patient with a proven catheter-related bloodstream infection (31, 32, 33, 34). It is homogeneously oxacillin resistant and has a 100% infection rate in the rat model used (34).

### Gene identification.

The sequences of the genes studied were retrieved from the National Center for Biotechnology Information (NCBI) GenBank (Table 1). On the basis of the sequences, primers were designed with Primer Express 2.0 software (Applied Biosystems Division of Perkin-Elmer) and used to amplify part of the corresponding genes in *S. epidermidis* 10b. The following thermal cycling conditions were used: 5 min at 95°C, followed by 25 repeats of 30s at 95°C, 30s at related annealing temperatures (Table 1) and 1 min at 72°C followed by 7 min at 72°C and holding at 4°C. PCR was performed on a GeneAmp PCR System 9700 (Perkin-Elmer Applied Biosystems, Foster City, Calif.). All primers and probes were provided by Eurogentec (Seraing, Belgium).

**Table1. sequences of primers (5'→3'), annealing temperature, fragment length and number of copies of gene per µL plasmid solution.**

| Genes | Forward primer | Reverse primer | Annealing Temperature °C) | Fragment Length (base pair) | Number of gene copies per µl of plasmid solution |
|---|---|---|---|---|---|
| SE1501 | CCAATTACTAGTATT AAATTCAG | CTACACTGTTAGA CGTGAG | 52 | 355 | 1.93 × 10¹⁰ |
| SE2232 | GTTGATAACCGTCAA CAAGG | CATGTTGATCTTTT GAATCCC | 62 | 388 | 1.51 × 10¹⁰ |

### Plasmid cloning and quantification of number of copies of the plasmid for Taqman quantitative PCR.

All genes were cloned as described elsewhere (31, 35). Pure plasmid DNA was obtained and quantified with the GeneQuant RNA/DNA calculator (Amersham Pharmacia Biotech, Uppsala, Sweden).

### Gene expression in in vitro bacteria.

The in vitro model has been described previously. Briefly, a frozen culture of *S*. *epidermidis* 10b was grown to the end -exponential growth phase, pelleted, and resuspended in 0.9% NaCl. Fragments (length; 7 mm) of a commercial polyurethane intravenous catheter (Arrow International, Reading, Pa.) were added to this bacterial suspension and placed in a water bath at 37°C. After different incubation periods, nucleic acids (RNA and DNA) were extracted instantaneously by an adaptation of the method of Cheung et al. (36) as previously described (31, 32, 33). Briefly, for bacteria in suspension (planktonic bacteria), an aliquot of a bacterial suspension was rapidly cooled on ice. The bacteria were pelleted, and the pellet was suspended in 500 µl of NAES buffer (50 mM sodium acetate [pH 5.1], 10 mM EDTA, 1% sodium dodecyl sulfate) and added to a FastRNA blue tube (Bio 101, Carlsbad, Calif.) with 500 µl acidified phenol-chloroform (5:1; pH 4.5; Ambion, Austin, Tex.) at room temperature. For the bacteria that adhered to a catheter segment, the colonized catheter fragment was washed with 1 ml of 0.9% NaCl and added directly to a FastRNA tube containing 500 µl of NAES buffer and 500 µl of acidified phenol-chloroform. The FastRNA tubes were shaken for 23 s at 6,000 rpm with a FastPrep instrument (FP 120; Bio 101, Savant, Holbrook, N.Y). After shaking, the tubes were centrifuged, and 90% of the supernatant (450µl) was precipitated with 520 µl of isopropyl alcohol and 35 µl of 3 M sodium acetate. The pellet was washed with 70% ethanol and resuspended in 150 µl of RNase-free water. Fifty microliters of this sample was diluted 1/10 and used for quantification of gDNA. The remaining 100 µl was purified with an Rneasy mini kit (Qiagen, Hilden, Germany) and treated with RNase-free DNase (Qiagen) on Rneasy columns according to the instructions of the manufacturer. The RNA was finally dissolved in 60 µl of RNase-free water. Reverse transcription of the RNA was performed with Moloney murine leukemia virus reverse transcriptase (Promega, Madison, Wisconsin, USA) as previously described (31, 35, 37). Nucleic acid isolation was done at time point 0 min (n = 6) (just before suspending the bacteria in 0.9% NaCl) and at time point 10 (n = 12), 35 (n = 12), 60 (n = 12), 120 (n = 12), and 180 (n = 12) min, both from the bacteria adherent to the catheters and from planktonic bacteria. Data for all time points were generated in six independent measurements.

### Gene expression in sessile bacteria during in vivo foreign body infection

We used a previously described model with some modifications in which first-generation descendants of inbred Fisher rats harbored under germfree conditions since 1965 were used (34). The rats used in our experiments were exposed to normal rat flora from birth and were labeled ex-germfree Fisher rats. In this model, catheter fragments were inoculated with a small amount of *S. epidermidis* 10b before implantation, which resulted in a 100% infection rate (34). The experimental conditions have been described in detail previously (31, 32, 33). Briefly, 7-mm catheter fragments were incubated for 20 min in a suspension of *S. epidermidis* in 0.9% NaCl as described above. After 20 min, the suspension with catheter fragments was placed on ice. Anesthesia of rats was induced with urethane and during the procedure the rats were kept anaesthetized with a combination of 20 % urethane and 80% oxygen. The back of each rat was shaved over a large area, and the skin was generously disinfected with 0.5% chlorhexidine in 70% alcohol and allowed to dry for 2 min. A 10-mm skin incision was made at the base of the tail of each adult ex-germfree Fisher rat, and the subcutis was carefully dissected to create four subcutaneous tunnels. In each rat, eight catheter fragments were quickly inserted subcutaneously at least 2 cm from the incision; the distance between two fragments was at least 1 cm. For catheter explantation, animals were sacrificed by using 0.5% CO₂. The skin was disinfected as described above, and the naked catheter segments were gently removed from the subcutaneous tissue. All catheter fragments from one animal were used for one time point. In each experiment, baseline expression levels in sessile bacteria before implantation were determined (time zero; *n* = 16). A total of 192 polyurethane catheter segments were implanted subcutaneously in the rat model, and these segments were explanted at 11 different time-points. The time-points used were 15 min (*n* = 16), 1 h (*n* = 16), 2 h (*n* = 16), 4 h (*n* = 16), 6 h (*n* = 16), 12 h (*n* = 16), 24 h (*n* = 16), 2 days (*n* = 16), 4 days (*n* = 16), 7 days (*n* = 16), and 14 days (*n* = 16). Data for each in vivo time point were generated in sixteen independent measurements generated in two independent experiments. Nucleic acid isolation and cDNA synthesis were performed immediately after explantation as described above.

### Taqman quantitative PCR.

Quantification of both cDNA and gDNA was done on the ABI Prism 7700 Sequence Detection System (PE Applied Biosystems), as described elsewhere (31, 35). In brief, quantitative PCR was done with 2 µL of cDNA, 12.5 µL of 2× Taqman PCR master mix (PE Applied Biosystems), 900 nmol/L of each primer, and 200 nmol/L probe in a final volume of 25 µL. Thermal cycling conditions were the following: 2 min at 50°C, followed by 10 min at 95°C, followed by 45 repeats of 15 s at 95°C and 1 min at 60°C. Data collection was done during each annealing phase. During each run, a standard dilution of the plasmid with known quantity was included to permit gene quantification by means of the supplied software, according to the instructions of the manufacturer. In each run, a negative control (distilled water) was included. The primers and probes for the genes are summarized in Table 2.

### Statistical analysis.

All statistical analyses were performed with Graphpadprism (Graphpad software version 4.2, San Diego, USA). Since the in vitro and in vivo cDNA/gDNA ratios were not normally distributed at any time point, all data were log₁₀ transformed in order to fulfil the requirements of normality.

**Table2. Taqman primers and probes (5'→3') used to study the expression of genes with putative roles in the pathogenesis of Staphylococcus epidermidis foreign body infections.**

| Genes | PID | Forward primer | Probe: labeled FAM-5' and 3'-TAMRA | Reverse primer |
|---|---|---|---|---|
| SE1501 | 27468419 | CCCTAAAGATGAAA CTACTGTTCATGAT | ACAATGTGACAAAACAGGG TGTACTAAAAGTAAACG | TGCTATCCATTCGAAAC TTTCATTAT |
| SE2232 | 27469150 | AGCATCACCATCTAA TAAAAACGAAA | TAACAAAGAAGAATCTAGT ACGACAACAAATCAATCCG A | CCATCATTACTTTTATC GTCTTTACTATCAC |

For the in vitro data, two hypotheses were tested. A significant change in gene expression levels over time within one group (sessile or planktonic) was tested with a one-way analysis of variance (One-way ANOVA). A significant difference in the evolution over time of the gene expression levels between the sessile group and the planktonic group was tested with a two-way ANOVA. When the one-way ANOVA was significant, two-sided univariate tests with a correction for multiple comparisons were done (Bonferroni test) to locate the significant differences.
For the in vivo data, a one-way ANOVA was used to test if there was a significant evolution of the expression levels over time. When the one-way ANOVA test was significant, the two-sided Bonferroni multiple-comparison method was used to determine which time points differed at a = 0.05, with a correction for multiple comparisons.

### Expression of the SE1501 gene in vitro in 0.9% NaCl.

In planktonic bacteria, expression of this gene decreased after inoculation and stayed at a low level throughout the observation period. In sessile bacteria, gene expression rapidly increased and remained at a high level.
The difference between expression in sessile and planktonic bacteria was highly significant (*P* < 0.0001, as determined by two-way ANOVA). The maximum difference in expression was reached at 60 min after inoculation (1.844 log₁₀ or 70-fold difference) and stayed almost constant till the end of the experiment (Fig.1.A).

### Expression of the SE1501 gene In vivo.

After implantation of the catheter fragments in the rats, expression in the sessile bacteria rapidly increased and peaked after 4 h (65-fold increase (95% CI of diff. -2.688 log₁₀ to -0.9352 log₁₀; *P* <0.001, as determined by a Bonferroni test) followed by a 26-fold decrease of gene expression in the next 20 hours. From 24 till 96 hours after implantation, a slight increase was observed which peaked at 168 h (one week) after implantation and subsequently decreased to the same level of gene expression observed at 24 h (Fig.2).

### Expression of the SE2232 gene in vitro in 0.9% NaCl..

In sessile and planktonic bacteria, expression of this gene decreased 10-fold during the first 35 min after inoculation in 0.9% NaCl (*P* <0.01, one-way ANOVA) and then remained almost constant till the end at 180 min in planktonic bacteria. In sessile bacteria, expression of this gene increased 13-fold from 35 min to 60 min and then remained constant during the 3 h observation period of the experiment (Fig. 1.B). The gene expression difference over time between planktonic and sessile was not significant

### Expression of the SE2232 gene in vivo.

Implantation induced a 1.321 log₁₀ or 21-fold increase in gene expression that peaked at 4 h (1.47 log₁₀ or 30-fold increase, 95% CI of diff. -2.170 log₁₀ to - 0.7704 log₁₀; P <0.001, as determined by a Bonferroni test) followed by a 1.941 log₁₀ or 87-fold decrease of gene expression in the next 20 hours. After 24 hrs, gene expression again slowly increased, peaked again at 168 hours (one week) after implantation and from then on, slowly decreased till the end at 336 h (*P*< .0001, 1-way ANOVA) (Fig.2).

Analysis of gene expression of the two genes (SE1501 and SE2232) selected for this study over time in our *in vivo* and *in vitro* models and FACS analysis and microscopy data (see Example 2) show that these two proteins are expressed in *S. epidermidis* 10b and are located at least partially on the surface of bacterial cells. The pattern of changes of gene expression over time in *in vitro* experiments is similar to the pattern of protein expression changes at relevant time points *in vitro* as confirmed by fluorescence microscopy data.
Gene expression of SE1501 slowly increased after exposure of bacteria to the foreign body and peaked 1 hour after inoculation in *vitro* and stayed high and constant after that. There is significant difference between the gene expression of SE1501 in sessile and planktonic bacteria. Early in vivo FBI data also showed an increase in gene expression after implantation. Microscopical images show that this protein is more expressed in sessile cells than planktonic bacteria (Fig.3A). Since this gene is absent in the biofilm producing strain *S*. *epidermidis* RP62A (28) and since polyclonal antibodies against SE1501 didn't show any biofilm inhibition effect (our data), the role of protein might be indirect (e.g., as a signal transduction factor).
The SE2232 gene exhibits some similarity to fibronectin binding proteins such as atlC and AAS found in other Staphylococcal species which their roles in biofilm formation were proven previously. The *in vivo* and *in vitro* expression of this gene did not show any changes during the first hour. No significant difference in expression of the SE2232 gene between sessile and planktonic bacteria was observed, although from the gene expression curve and microscopy images it can be concluded that the expression of this protein in sessile bacteria is slightly higher than in planktonic bacteria (Fig.3B). However, both SE1501 and SE2232 gene expression during late in vivo FBI were high.

### Example 2. Production of antibodies

### Cloning, expression and purification of recombinant proteins.

We amplified the genes for production of the recombinant proteins by PCR with primers of SE1501F (5' CACGTGCTAGCGCTGAATCAAACACTTCAGTTTCTTCT 3'), SE1501R (5'ATGCGGATCCTAGTGATGGTGATGGTGATGCATACCTGTATTTGGTAAT AG 3'), SE2232F (5' ACGTGCTAGCGCAGATTCAGAAAGTACATC 3') and SE2232R (5'ATGCGGATCCTAGTGATGGTGATGGTGATGATCAGCTGTAGCTGTTCC 3'), which incorporate flanking *Nhe*I and *Bam*HI restriction sites and sequence coding for a C-terminal His⁶ tag. The genes were cloned into a pET11c expression vector (Stratagene, LaJolla, CA) and we electrotransformed the recombinant plasmids into *E. coli* BL21 (DE3). The expression of recombinant protein was induced by the addition of IPTG, and the recombinant proteins were purified by Ni⁺ affinity chromatography with the HisTrap^{™} Kit (Amersham Pharmacia, Uppsala, Sweden) according to the manufactures recommendations. We determined the purity of the recombinant proteins by Coomassie Blue staining of SDS-PAGE gel electrophoresis. The sequence of the purified peptides was confirmed with MALDI-TOF mass spectrometry.

### Production of recombinant protein rabbit antiserum.

Rabbit immunization with each recombinant protein was done by Eurogentec (Seraing, Belgium), according to standard immunization protocols.

### Purification of antibodies:

Sera from pre and post-immunization rabbits were passed onto a protein-G sepharose column (Pharmacia) equilibrated against PBS. After washing the column with PBS, bound IgG's were eluted using 0.1 M Glycin-HCl pH 2.7. The eluate was dialysed extensively against PBS and the IgG content was measured spectrophotometrically (OD₂₈₀ₙₘ, ε: 1.4).

### Flow cytometry analysis and Fluorescence microscopy.

Preparation of samples for FACS analysis and fluorescence microscopy was similar to preparation of samples for gene expression analysis *in vitro* with the exception that here samples were taken at different time points (0, 30, 60, 90 and 120 min) of bacterial growth and planktonic cells were also sonicated using an ultrasound bath (Branson 2510), in a manner similar to that used to separate the sessile cells from the catheters. Then the bacteria were fixed using PBS containing 3% formaldehyde and 1% glutaraldehyde for 30 min to 1 hour, after which the cells were washed with PBS. The number of bacteria per ml was estimated by measuring the OD₆₀₀ₙₘ and the bacterial suspensions were subsequently diluted to an OD₆₀₀ₙₘ of 0.05 (~5 x 10⁷ cells) in PBS. Purified rabbit antibodies were added at 5 µg/ 100µl to the bacterial solution which was then incubated for 30 minutes on ice. Afterwards the bacteria were washed twice with PBS and FITC-labeled goat-anti-rabbit (BD-Pharmingen) was added for 20-30 min. The cells were washed twice with PBS prior to analyzing via FACS vantage (Beckton Dickinson).
The rabbit antibodies used were: RPAbSE1501 and RPAbSE2232 and a control rabbit IgG (polyclonal rabbit antibody against mouse immunoglobins). A total of 10,000 bacteria were counted and analyzed with CellQuest software (BD Biosciences).
Five µl of the solution was applied to a microscope slide and fixed with a cover glass. Cells were viewed with a fluorescence microscope (Leica, Germany) equipped with an oil-immersion plan neofluar objective (100X; numeric aperture = 1.25), digital images were obtained by using corresponding filter and camera
Cells plus control rabbit IgG and FITC-labeled goat-anti-rabbit, cells plus FITC-labeled goat-anti-rabbit and cells plus respectively RPAbSE1501 or RPAbSE2232 were used as negative controls in FACS and microscopy analysis assays (Fig. 3).

### FACS analysis.

According to data obtained by FACS analysis, the shift in fluorescence intensity was similar between sessile and planktonic cells. These results are in contradiction with the gene expression data, that showed a higher level of expression of these proteins in sessile bacteria. Because this discrepancy between the FACS results and gene-expression data could be due to aggregation of sessile bacteria, we used microscope analysis to visualize the cells. We observed that sonification of sessile bacteria, even for 10 min, would not completely separate the bacteria in sessile form. Moreover, in the FACS analysis, the size effect (aggregation of cells) plays a role in the final result. In this context, it is important to mention that we observed a shift in the amount of cells from the area that cells were gated to calculate the mean of the peak toward the area of bigger particles size in sessile samples (data not shown).

### Fluorescence microscopy and protein expression

Comparison of the images taken by fluorescence microscope from sessile and planktonic bacteria at different time points using RPAbSE1501 and RPAbSE2232 antibodies and FITC-labelled goat-anti-rabbit antibody confirmed the higher levels of gene expression in the sessile form (Fig. 3). To confirm that SE1501 and SE2232 proteins were localized at least partially on the surface of bacteria, bacteria from time point zero samples were incubated with primary antibodies (RPAbSE1501 or RPAbSE2232), fixed and then incubated with FITC-labelled goat-anti-rabbit antibody. The results obtained from live or fixed cells at time point zero were similar. Antibodies, being large proteins, cannot pass through intact cellular or subcellular membranes in living cells (39). Hence, it can be concluded that these two proteins are mainly located on the cell surface of bacteria.

### Example 3 Biofilm inhibition

### Biofilm inhibition assay.

The amount of biofilm formed was determined by a semiquantitive microtiter plate method as described (18, 38). A frozen culture of *S. epidermidis* 10b was grown to the end-exponential growth phase, pelleted, and resuspended in 0.9% NaCl, diluted to an OD₆₀₀ of 0.05 (~5 x 10⁷ cells) and mixed with polyclonal antibodies. Sixplicate samples (each containing 0.1 ml) were added to individual wells of a 96-well flat-bottom microwell plate (comp). Microwell plates were incubated 2 h at 4°C and then overnight at 37°C. After three washes with PBS, any remaining biofilm was stained with safranin O dye for 1 min and washed with PBS again. Optical density at 492 nm (OD₄₉₂) was determined with a 96-well plate spectrometer reader. Percent inhibition of biofilm formation was calculated by using the following formula: (*A*_{492, positive} - *A*_{492, antibody})/(*A*_{492, positive} - *A*_{492, negative}) × 100%. (18). 0.9% NaCl medium without bacteria was used as negative control.
For the biofilm inhibition data, two hypotheses were tested. A significant change in biofilm inhibition levels over different concentrations of IgG's within one group (pre or post-immunization) was tested with a one-way analysis of variance (One-way ANOVA). A significant difference in the evolution over different concentrations of IgG's of the biofilm inhibition levels between the pre-immunization group and post-immunization group was tested with a two-way ANOVA. When the one-way ANOVA was significant, two-sided univariate tests with a correction for multiple comparisons were done (Bonferroni test) to locate the significant differences.

### Biofilm inhibition by polyclonal antibodies (Fig. 4).

Different concentrations of purified IgG's from pre and post-immunization sera of rabbits immunized against both SE1501 and SE2232 recombinant proteins were used to study the effect on biofilm formation. Increasing concentrations of polyclonal RPAbSE2232 antibodies led to increasing inhibition of biofilm formation. At a concentration of 30µg/ml, biofilm inhibition by RPAbSE2232 reached a maximum of 80%, whereas biofilm inhibition obtained by different concentrations of the IgG fraction of pre-immune rabbit sera was constant (≈30%).
Biofilm inhibition by the IgG fraction purified from pre-immune sera of rabbits subsequently immunized against SE1501 was higher than that obtained by the post-immunization IgG fraction. At a concentration of 20µg/ml, biofilm inhibition by pre-immune IgG reached a maximum of 90%, whereas for the post-immunization IgG fraction, a maximum biofilm inhibition of 75% was obtained at a concentration of 40µg/ml. The 75% biofilm inhibition obtained at the concentration of 40 µg/ml of post-immunization IgG is probably not a specific effect of anti-SE1501 IgG's but mainly due to antibodies against other antigens which exist in pre-immune rabbit sera.

## Claims

1. Antibody against protein SE2232 of *Staphylococcus epidermis* or a homologous protein thereof

2. Antibody according to claim 1, selected from the group consisting of polyclonal antibodies, monoclonal antibodies, single chain antibodies, humanised antibodies and SE2232 binding antibody fragments such as F(ab), F(ab)2 and scFV.

3. Antibody according to claim 1, where the homologous protein is chosen from the proteins listed in Figure 5.

4. A method for preventing biofilm formation by *Staphylococcus* on a medical device comprising coating said medical device with an antibody according to claim 1 or 2.

5. A method according to claim 4, wherein said medical device is an intravascular device such as a stent or a cannula, a prosthesis, such as an orthopaedic endoprothesis or a prosthetic valve, a pacemaker, a catheter, a drain, an endovascular graft or a cerebrovascular shunt.

6. A method according to claim 4 or 5, wherein said coated medical device is implanted or inserted into a mammalian body, preferably a human body.

7. Immunogenic composition comprising protein SE2232 of *Staphylococcus epidermis,* a homologous protein thereof, or an immunological active fragment thereof.

8. Vaccine composition comprising the immunogenic composition of claim 7 and a pharmaceutically acceptable carrier.

9. A method for preventing biofilm formation in a mammal by administering an antibody according to any of the claims 1-3 or a vaccine composition according to claim 8 to a subject in need thereof.

10. A method according to claim 9, wherein said method comprises a prophylactic or a therapeutical treatment.

11. Use of an antibody according to any of claims 1-3or a vaccine composition according to claim 8 for preventing biofilm formation.

12. Use according to claim 11, wherein the antibody is coated onto a medical device.


SEQUENCE LISTING

<110> Katholieke Universiteit Leuven
      Stichting Katholieke Universiteit, more particularly the
      Radboud University Nijmegen Medical Centre

<120> Prevention of Staphylococcus biofilm formation

<130> P80212EP00

<140> EP 07106164.2
<141> 2007-04-13

<160> 32

<170> PatentIn version 3.3

<210> 1
<211> 5
<212> PRT
<213> Artificial

<220>
<223> LPxTG motif


<220>
<221> misc_feature
<222> (3)..(3)
<223> Xaa can be any naturally occurring amino acid

<400> 1

Leu Pro Xaa Thr Gly
1               5


<210> 2
<211> 15
<212> PRT
<213> Artificial

<220>
<223> linker 1

<400> 2

Gly Gly Gly Gly Ser Gly Gly Gly Gly Ser Gly Gly Gly Gly Ser
1               5                   10                  15


<210> 3
<211> 15
<212> PRT
<213> Artificial

<220>
<223> linker 2

<400> 3

Glu Ser Gly Arg Ser Gly Gly Gly Gly Ser Gly Gly Gly Gly Ser
1               5                   10                  15


<210> 4
<211> 14
<212> PRT

<213>  Artificial

<220>
<223>  linker 3

<400>  4

Glu Gly Lys Ser Ser Gly Ser Gly Ser Glu Ser Lys Ser Thr
1               5                   10


<210>  5
<211>  15
<212>  PRT
<213>  Artificial

<220>
<223>  linker 4

<400>  5

Glu Gly Lys Ser Ser Gly Ser Gly Ser Glu Ser Lys Ser Thr Gln
1               5                   10                  15


<210>  6
<211>  14
<212>  PRT
<213>  Artificial

<220>
<223>  linker 5

<400>  6

Glu Gly Lys Ser Ser Gly Ser Gly Ser Glu Ser Lys Val Asp
1               5                   10


<210>  7
<211>  14
<212>  PRT
<213>  Artificial

<220>
<223>  linker 6

<400>  7

Gly Ser Thr Ser Gly Ser Gly Lys Ser Ser Glu Gly Lys Gly
1               5                   10


<210>  8
<211>  18
<212>  PRT
<213>  Artificial

<220>
<223>  linker 7

<400>  8

Lys Glu Ser Gly Ser Val Ser Ser Glu Gln Leu Ala Gln Phe Arg Ser
1               5                   10                  15


Leu Asp

<210>  9
<211>  16
<212>  PRT
<213>  Artificial

<220>
<223>  linker 8

<400>  9

Glu Ser Gly Ser Val Ser Ser Glu Glu Leu Ala Phe Arg Ser Leu Asp
1               5                   10                  15


<210>  10
<211>  23
<212>  DNA
<213>  Artificial

<220>
<223>  Fd primer 1501

<400>  10
ccaattacta gtattaaatt cag                                              23


<210>  11
<211>  19
<212>  DNA
<213>  Artificial

<220>
<223>  Rv primer 1501

<400>  11
ctacactgtt agacgtgag                                                   19


<210>  12
<211>  20
<212>  DNA
<213>  Artificial

<220>
<223>  Fd primer 2232

<400>  12
gttgataacc gtcaacaagg                                                  20


<210>  13
<211>  21
<212>  DNA
<213>  Artificial

<220>
<223>  Rv primer 2232

<400>  13
catgttgatc ttttgaatcc c                                                21


<210>  14
<211>  28
<212>  DNA

<213>  Artificial

<220>
<223>  TFd primer 1501

<400>  14
ccctaaagat gaaactactg ttcatgat                                          28


<210>  15
<211>  36
<212>  DNA
<213>  Artificial

<220>
<223>  1501 probe

<400>  15
acaatgtgac aaaacagggt gtactaaaag taaacg                                 36


<210>  16
<211>  26
<212>  DNA
<213>  Artificial

<220>
<223>  TRv primer 1501

<400>  16
tgctatccat tcgaaacttt cattat                                            26


<210>  17
<211>  26
<212>  DNA
<213>  Artificial

<220>
<223>  TFd primer 2232

<400>  17
agcatcacca tctaataaaa acgaaa                                            26


<210>  18
<211>  39
<212>  DNA
<213>  Artificial

<220>
<223>  2232 probe

<400>  18
taacaaagaa gaatctagta cgacaacaaa tcaatccga                              39


<210>  19
<211>  31
<212>  DNA
<213>  Artificial

<220>
<223>  TRv primer 2232

<400>  19
ccatcattac ttttatcgtc tttactatca c                                      31

<210>  20
<211>  38
<212>  DNA
<213>  Artificial

<220>
<223>  SE1501F primer

<400>  20
cacgtgctag cgctgaatca aacacttcag tttcttct                                38


<210>  21
<211>  51
<212>  DNA
<213>  Artificial

<220>
<223>  SE1501R primer

<400>  21
atgcggatcc tagtgatggt gatggtgatg catacctgta tttggtaata g                 51


<210>  22
<211>  30
<212>  DNA
<213>  Artificial

<220>
<223>  SE2232F primer

<400>  22
acgtgctagc gcagattcag aaagtacatc                                         30


<210>  23
<211>  48
<212>  DNA
<213>  Artificial

<220>
<223>  SE2232R primer

<400>  23
atgcggatcc tagtgatggt gatggtgatg atcagctgta gctgttcc                     48


<210>  24
<211>  676
<212>  PRT
<213>  Staphylococcus epidermidis

<400>  24

Met Lys Arg Thr Asp Lys Ile Gly Val Tyr Leu Lys Leu Ser Cys Ser
1               5                   10                  15


Ala Leu Leu Leu Ser Gly Ser Leu Val Gly Tyr Gly Phe Thr Lys Asp
            20                  25                  30


Ala Phe Ala Asp Ser Glu Ser Thr Ser Ser Asn Val Glu Asn Thr Ser
        35                  40                  45

Asn Ser Asn Ser Ile Ala Asp Lys Ile Gln Gln Ala Lys Asp Asp Ile
    50                  55                  60

Lys Asp Leu Lys Glu Leu Ser Asp Ala Asp Ile Lys Ser Phe Glu Glu
65                  70                  75                  80

Arg Leu Asp Lys Val Asp Asn Gln Ser Ser Ile Asp Arg Ile Ile Asn
                85                  90                  95

Asp Ala Lys Asp Lys Asn Asn His Leu Lys Ser Thr Asp Ser Ser Ala
            100                 105                 110

Thr Ser Ser Lys Thr Glu Asp Asp Asp Thr Ser Glu Lys Asp Asn Asp
        115                 120                 125

Asp Met Thr Lys Asp Leu Asp Lys Ile Leu Ser Asp Leu Asp Ser Ile
    130                 135                 140

Ala Lys Asn Val Asp Asn Arg Gln Gln Gly Glu Asn Ser Ala Ser Lys
145                 150                 155                 160

Pro Ser Asp Ser Thr Thr Asp Glu Lys Asp Asp Ser Asn Asn Lys Val
                165                 170                 175

His Asp Thr Asn Ala Ser Thr Arg Asn Ala Thr Thr Asp Asp Ser Glu
            180                 185                 190

Glu Ser Val Ile Asp Lys Leu Asp Lys Ile Gln Gln Asp Phe Lys Ser
        195                 200                 205

Asp Ser Asn Asn Lys Leu Ser Glu Gln Ser Asp Gln Gln Ala Ser Pro
    210                 215                 220

Ser Asn Lys Asn Glu Asn Asn Lys Glu Glu Ser Ser Thr Thr Thr Asn
225                 230                 235                 240

Gln Ser Asp Ser Asp Ser Lys Asp Asp Lys Ser Asn Asp Gly Arg Arg
                245                 250                 255

Ser Thr Leu Glu Arg Ile Ala Ser Asp Thr Asp Gln Ile Arg Asp Ser
            260                 265                 270

Lys Asp Gln His Val Thr Asp Glu Lys Gln Asp Ile Gln Ala Ile Thr
        275                 280                 285

Arg Ser Leu Gln Gly Ser Asp Lys Ile Glu Lys Ala Leu Ala Lys Val
    290                 295                 300

Gln Ser Asp Asn Gln Ser Leu Asp Ser Asn Tyr Ile Asn Asn Lys Leu
305                 310                 315                 320

Met Asn Leu Arg Ser Leu Asp Thr Lys Val Glu Asp Asn Asn Thr Leu
                325                 330                 335

Ser Asp Asp Lys Lys Gln Ala Leu Lys Gln Glu Ile Asp Lys Thr Lys
            340                 345                 350

Gln Ser Ile Asp Arg Gln Arg Asn Ile Ile Ile Asp Gln Leu Asn Gly
        355                 360                 365

Ala Ser Asn Lys Lys Gln Ala Thr Glu Asp Ile Leu Asn Ser Val Phe
    370                 375                 380

Ser Lys Asn Glu Val Glu Asp Ile Met Lys Arg Ile Lys Thr Asn Gly
385                 390                 395                 400

Arg Ser Asn Glu Asp Ile Ala Asn Gln Ile Ala Lys Gln Ile Asp Gly
                405                 410                 415

Leu Ala Leu Thr Ser Ser Asp Asp Ile Leu Lys Ser Met Leu Asp Gln
            420                 425                 430

Ser Lys Asp Lys Glu Ser Leu Ile Lys Gln Leu Leu Thr Thr Arg Leu
        435                 440                 445

Gly Asn Asp Glu Ala Asp Arg Ile Ala Lys Lys Leu Leu Ser Gln Asn
    450                 455                 460

Leu Ser Asn Ser Gln Ile Val Glu Gln Leu Lys Arg His Phe Asn Ser
465                 470                 475                 480

Gln Gly Thr Ala Thr Ala Asp Asp Ile Leu Asn Gly Val Ile Asn Asp
                485                 490                 495

Ala Lys Asp Lys Arg Gln Ala Ile Glu Thr Ile Leu Gln Thr Arg Ile
            500                 505                 510

Asn Lys Asp Lys Ala Lys Ile Ile Ala Asp Val Ile Ala Arg Val Gln
        515                 520                 525

Lys Asp Lys Ser Asp Ile Met Asp Leu Ile His Ser Ala Ile Glu Gly
    530                 535                 540

Lys Ala Asn Asp Leu Leu Asp Ile Glu Lys Arg Ala Lys Gln Ala Lys
545                 550                 555                 560

Lys Asp Leu Glu Tyr Ile Leu Asp Pro Ile Lys Asn Arg Pro Ser Leu
                565                 570                 575

Leu Asp Arg Ile Asn Lys Gly Val Gly Asp Ser Asn Ser Ile Phe Asp
            580                 585                 590

Arg Pro Ser Leu Leu Asp Lys Leu His Ser Arg Gly Ser Ile Leu Asp
        595                 600                 605

Lys Leu Asp His Ser Ala Pro Glu Asn Gly Leu Ser Leu Asp Asn Lys
    610                 615                 620

Gly Gly Leu Leu Ser Asp Leu Phe Asp Asp Asp Gly Asn Ile Ser Leu
625                 630                 635                 640

Pro Ala Thr Gly Glu Val Ile Lys Gln His Trp Ile Pro Val Ala Val
                645                 650                 655

Val Leu Met Ser Leu Gly Gly Ala Leu Ile Phe Met Ala Arg Arg Lys
            660                 665                 670

Lys His Gln Asn
        675

<210> 25
<211> 676
<212> PRT
<213> Staphylococcus epidermidis

<400> 25

Met Lys Arg Thr Asp Lys Ile Gly Val Tyr Leu Lys Leu Ser Cys Ser
1               5                   10                  15

Ala Leu Leu Leu Ser Gly Ser Leu Val Gly Tyr Gly Phe Thr Lys Asp
            20                  25                  30

Ala Phe Ala Asp Ser Glu Ser Thr Ser Ser Asn Val Glu Asn Thr Ser
        35                  40                  45

Asn Ser Asn Ser Ile Ala Asp Lys Ile Gln Gln Ala Lys Asp Asp Ile
    50                  55                  60

Lys Asp Leu Lys Glu Leu Ser Asp Ala Asp Ile Lys Ser Phe Glu Glu
65                  70                  75                  80

Arg Leu Asp Lys Val Asp Asn Gln Ser Ser Ile Asp Arg Ile Ile Asn
                85                  90                  95

Asp Ala Lys Asp Lys Asn Asn His Leu Lys Ser Thr Asp Ser Ser Ala
            100                 105                 110

Thr Ser Ser Lys Thr Glu Asp Asp Asp Thr Ser Glu Lys Asp Asn Asp
        115                 120                 125

Asp Met Thr Lys Asp Leu Asp Lys Ile Leu Ser Asp Leu Asp Ser Ile
    130                 135                 140

Ala Lys Asn Val Asp Asn Arg Gln Gln Gly Glu Glu Arg Ala Ser Lys

145                 150                 155                 160

Pro Ser Asp Ser Thr Thr Asp Glu Lys Asp Asp Ser Asn Asn Lys Val
                165                 170                 175

His Asp Thr Asn Ala Ser Thr Arg Asn Ala Thr Thr Asp Asp Ser Glu
            180                 185                 190

Glu Ser Val Ile Asp Lys Leu Asp Lys Ile Gln Gln Asp Phe Lys Ser
        195                 200                 205

Asp Ser Asn Asn Asn Pro Ser Glu Gln Ser Asp Gln Gln Ala Ser Pro
    210                 215                 220

Ser Asn Lys Thr Glu Asn Asn Lys Glu Glu Ser Ser Thr Thr Thr Asn
225                 230                 235                 240

Gln Ser Asp Ser Asp Ser Lys Asp Asp Lys Ser Asn Asp Gly His Arg
                245                 250                 255

Ser Thr Leu Glu Arg Ile Ala Ser Asp Thr Asp Gln Ile Arg Asp Ser
            260                 265                 270

Lys Asp Gln His Val Thr Asp Glu Lys Gln Asp Ile Gln Ala Ile Thr
        275                 280                 285

Arg Ser Leu Gln Gly Ser Asp Lys Ile Glu Lys Ala Leu Ala Lys Val
    290                 295                 300

Gln Ser Asp Asn Gln Ser Leu Asp Ser Asn Tyr Ile Asn Asn Lys Leu
305                 310                 315                 320

Met Asn Leu Arg Ser Leu Asp Thr Lys Val Glu Asp Asn Asn Thr Leu
                325                 330                 335

Ser Asp Asp Lys Lys Gln Ala Leu Lys Gln Glu Ile Asp Lys Thr Lys
            340                 345                 350

Gln Ser Ile Asp Arg Gln Arg Asn Ile Ile Ile Asp Gln Leu Asn Gly
        355                 360                 365

Ala Ser Asn Lys Lys Gln Ala Thr Glu Asp Ile Leu Asn Ser Val Phe
    370                 375                 380

Ser Lys Asn Glu Val Glu Asp Ile Met Lys Arg Ile Lys Thr Asn Gly
385                 390                 395                 400

Arg Ser Asn Glu Asp Ile Ala Asn Gln Ile Ala Lys Gln Ile Asp Gly
                405                 410                 415

Leu Ala Leu Thr Ser Ser Asp Asp Ile Leu Lys Ser Met Leu Asp Gln

420                 425                 430

Ser Lys Asp Lys Glu Ser Leu Ile Lys Gln Leu Leu Thr Thr Arg Leu
        435                 440                 445

Gly Asn Asp Glu Ala Asp Arg Ile Ala Lys Lys Leu Leu Ser Gln Asn
    450                 455                 460

Leu Ser Asn Ser Gln Ile Val Glu Gln Leu Lys Arg His Phe Asn Ser
465                 470                 475                 480

Gln Gly Thr Ala Thr Ala Asp Asp Ile Leu Asn Gly Val Ile Asn Asp
                485                 490                 495

Ala Lys Asp Lys Arg Gln Ala Ile Glu Thr Ile Leu Gln Thr Arg Ile
            500                 505                 510

Asn Lys Asp Lys Ala Lys Ile Ile Ala Asp Val Ile Ala Arg Val Gln
        515                 520                 525

Lys Asp Lys Ser Asp Ile Met Asp Leu Ile His Ser Ala Ile Glu Gly
    530                 535                 540

Lys Ala Asn Asp Leu Leu Asp Ile Glu Lys Arg Ala Lys Gln Ala Lys
545                 550                 555                 560

Lys Asp Leu Glu Tyr Ile Leu Asp Pro Ile Lys Asn Arg Pro Ser Leu
                565                 570                 575

Leu Asp Arg Ile Asn Lys Gly Val Gly Asp Ser Asn Ser Ile Phe Asp
            580                 585                 590

Arg Pro Ser Leu Leu Asp Lys Leu His Ser Arg Gly Ser Ile Leu Asp
        595                 600                 605

Lys Leu Asp His Ser Ala Pro Glu Asn Gly Leu Ser Leu Asp Asn Lys
    610                 615                 620

Gly Gly Leu Leu Ser Asp Leu Phe Asp Asp Asp Gly Asn Ile Ser Leu
625                 630                 635                 640

Pro Ala Thr Gly Glu Val Ile Lys Gln His Trp Ile Pro Val Ala Val
                645                 650                 655

Val Leu Met Ser Leu Gly Gly Ala Leu Ile Phe Met Ala Arg Arg Lys
            660                 665                 670

Lys His Gln Asn
        675

<210>  26

<211>  670
<212>  PRT
<213>  Staphylococcus haemolyticus

<400>  26
   Met Gly Tyr Gly Phe Thr Lys Asp Gly Phe Ala Gln Ser Asn Asp Arg
   1               5                   10                  15
   Ile Asp Asn Val Ser Ser Glu Met Thr Ser Val Gln Asn Lys Leu Asp
               20                  25                  30
   Lys Ala Ile Asp Lys Ala Lys Ala Lys Ile Asp Arg Leu Lys Tyr Leu
           35                  40                  45
   Gln Ala Thr Asp Ile Lys Ser Tyr Lys Glu Asp Ile Glu Asp Ala Arg
       50                  55                  60
   Asn Gln Ser Glu Ile Asp Gln Ile Leu Arg Asp Ala Gln Glu Glu Asp
   65                  70                  75                  80
   Arg Ile Ser Asn Glu Glu Ser Thr Lys Glu Thr Gly Glu Lys Ala Ser
                   85                  90                  95
   Thr Ser Asn Glu Ser Ser Leu Ser Thr Ala Lys Gln Ser Ser Thr Asn
               100                 105                 110
   Glu Glu Asn Lys Leu Asp Glu Leu Asp Lys Val Ile Ala Asp Leu Asp
           115                 120                 125
   Ser Leu Ser Glu Lys Val Asp Thr His Gln Gln Asn Gly Asp Met Lys
       130                 135                 140
   Ser Glu Gln Asp Ser Thr Asn Asn Asn Glu Asn Ser Gln Ile Ser Ser
   145                 150                 155                 160
   Gly Gln Ser Ala Ser Ser Gln Asn Asn Lys Asn Gln Ile Asn Asp Lys
                   165                 170                 175
   Asn Asp Asp Thr Ser Ile Leu Asp Glu Met Asp Asn Val Lys Asn Asp
               180                 185                 190
   Ile Glu Ser Thr Lys Glu Ser Ala His Ser Ser Val Glu Asp Ile Arg
           195                 200                 205
   Asp Gln Thr Asp Ser Ser Ile Gln Asp Asp Asn Ser Thr Glu Ser Gln
       210                 215                 220
   Ser Lys Asn Asn Thr Asn Thr Ala Ser Asp Lys Ser Ile Leu Ser Gly
   225                 230                 235                 240
   Ile Lys Gln Ile Asp Lys Asp Glu Asp Ser His Lys Ser Asn Lys Ile
                   245                 250                 255
   Asp Ser Lys Glu Gly His Val Asp Ala Leu Thr Asp Glu Leu Ser Ala
   260 265 270
   Asn Gln Lys Ile Asp Gln Ala Ile Thr Lys Val Glu Asn Gln Gln Asp
   275 280 285
   Asn Thr Ser Lys Arg Tyr Ser Asp His Lys Leu Glu Gln Leu Arg Gln
   290 295 300
   Leu Glu Gln Gln Val Lys Gln Asn Asn Asn Leu Thr Asn Glu Gln Lys
   305 310 315 320
   Gln Asn Val Glu Lys Asp Ile Lys Ile Val Arg Gln Asn Val Lys Ala
   325 330 335
   Asn Arg Asp Glu Ile Ser Gly Arg Leu Glu Gln Ser Ser Asn Lys Gln
   340 345 350
   Ala Thr Val Glu Gln Ile Leu Gly Ser Val Phe Ser Lys Asn Glu Ala
   355 360 365
   Gln Lys Ile Ala Lys Lys Ile Lys Thr Asn Gly Gln Ser Asp Lys Gln
   370 375 380
   Ile Thr Asp Gln Met Met Lys His Ile Asp Asn Leu Lys Thr Thr Thr
   385 390 395 400
   Ser Asp Asp Ile Leu Ala Ser Met Phe Asp Gln Ala Pro Asp Lys Glu
   405 410 415
   Ala Leu Ile Lys Thr Leu Leu Ser Thr Arg Leu Gly Asn Asn Glu Ala
   420 425 430
   Ser Gln Ile Ala Lys Gln Leu Ala Lys Glu Asn Leu Ser Ser Ser Glu
   435 440 445
   Leu Val Asn Gln Val Lys Gln Lys Ile Asn Ala Asn Gln Lys Ile Thr
   450 455 460
   Ala Asp Asp Ile Leu Lys Asp Val Leu Asp Lys Ser Ser Asp Pro Lys
   465 470 475 480
   Gln Thr Ile Glu Thr Leu Leu Ala Thr Lys Leu Asn Gln Thr Gln Ala
   485 490 495
   Lys Ala Leu Ala Asp Leu Ile Ala Arg Ala Gln Asn Asp Lys Ala Asn
   500 505 510
   Ala Leu Asp Leu Val Lys Asn Ala Leu Asn Gly Thr Ala Ser Asp Leu
   515 520 525
   Leu Gln Leu Gln Asn Lys Leu Asp Thr Ala Lys Asn Asn Leu Ser Tyr
       530                 535                 540
   Ile Leu Asp Pro Ile Thr Asn Arg Pro Ser Leu Phe Asp Arg Ile Asn
   545                 550                 555                 560
   Gly Ser Ala Ser Ser Ser Asn Ser Leu Asn Gln Gly Ser His Leu Leu
                   565                 570                 575
   Asp Gly Leu Thr Gly Ser Ser Leu Leu Asp Gly Leu Asn Ser Gly Gly
               580                 585                 590
   Ser Leu Leu Asp Asn Ile Glu Asp Ile Pro Asn Pro Val Gln Gly Leu
           595                 600                 605
   Ser Leu Gly Gln Leu Gly Asp Asp Asp Gly Phe Leu Ser Gly Leu Phe
       610                 615                 620
   Asp Asp Glu Gly Asn Leu Ser Leu Pro Asn Thr Gly Glu Val Val Lys
   625                 630                 635                 640
   Lys Ser Trp Leu Pro Val Thr Val Leu Leu Val Ile Ala Gly Gly Thr
                   645                 650                 655
   Leu Ile Gly Leu Gly Arg Arg Lys Gln Gln Lys Thr Lys Gln
               660                 665                 670

<210>   27
<211>   635
<212>   PRT
<213>   Staphylococcus aureus

<400>   27

Met Ala Lys Tyr Arg Gly Lys Pro Phe Gln Leu Tyr Val Lys Leu Ser
1               5                   10                  15

Cys Ser Thr Met Met Ala Thr Ser Ile Ile Leu Thr Asn Ile Leu Pro
            20                  25                  30

Tyr Asp Ala Gln Ala Ala Ser Glu Lys Asp Thr Glu Ile Thr Lys Glu
        35                  40                  45

Ile Leu Ser Lys Gln Asp Leu Leu Asp Lys Val Asp Lys Ala Ile Arg
    50                  55                  60

Gln Ile Glu Gln Leu Lys Gln Leu Ser Ala Ser Ser Lys Glu His Tyr
65                  70                  75                  80

Lys Ala Gln Leu Asn Glu Ala Lys Thr Ala Ser Gln Ile Asp Glu Ile
                85                  90                  95

Ile Lys Arg Ala Asn Glu Leu Asp Ser Lys Asp Asn Lys Ser Ser His
            100                 105                 110

Thr Glu Met Asn Gly Gln Ser Asp Ile Asp Ser Lys Leu Asp Gln Leu
        115                 120                 125

Leu Lys Asp Leu Asn Glu Val Ser Ser Asn Val Asp Arg Gly Gln Gln
    130                 135                 140

Ser Gly Glu Asp Asp Leu Asn Ala Met Lys Asn Asp Met Ser Gln Thr
145                 150                 155                 160

Ala Thr Thr Lys His Gly Glu Lys Asp Asp Lys Asn Asp Glu Ala Met
                165                 170                 175

Val Asn Lys Ala Leu Glu Asp Leu Asp His Leu Asn Gln Gln Ile His
            180                 185                 190

Lys Ser Lys Asp Ala Ser Lys Asp Thr Ser Glu Asp Pro Ala Val Ser
        195                 200                 205

Thr Thr Asp Asn Asn His Glu Val Ala Lys Thr Pro Asn Asn Asp Gly
    210                 215                 220

Ser Gly His Val Val Leu Asn Lys Phe Leu Ser Asn Glu Glu Asn Gln
225                 230                 235                 240

Ser His Ser Asn Arg Leu Thr Asp Lys Leu Gln Gly Ser Asp Lys Ile
                245                 250                 255

Asn His Ala Met Ile Glu Lys Leu Ala Lys Ser Asn Ala Ser Thr Gln
            260                 265                 270

His Tyr Thr Tyr His Lys Leu Asn Thr Leu Gln Ser Leu Asp Gln Arg
        275                 280                 285

Ile Ala Asn Thr Gln Leu Pro Lys Asn Gln Lys Ser Asp Leu Met Ser
    290                 295                 300

Glu Val Asn Lys Thr Lys Glu Arg Ile Lys Ser Gln Arg Asn Ile Ile
305                 310                 315                 320

Leu Glu Glu Leu Ala Arg Thr Asp Asp Lys Lys Tyr Ala Thr Gln Ser
                325                 330                 335

Ile Leu Glu Ser Ile Phe Asn Lys Asp Glu Ala Val Lys Ile Leu Lys
            340                 345                 350

Asp Ile Arg Val Asp Gly Lys Thr Asp Gln Gln Ile Ala Asp Gln Ile
        355                 360                 365

Thr Arg His Ile Asp Gln Leu Ser Leu Thr Thr Ser Asp Asp Leu Leu
    370                 375                 380

Thr Ser Leu Ile Asp Gln Ser Gln Asp Lys Ser Leu Leu Ile Ser Gln
385                 390                 395                 400

Ile Leu Gln Thr Lys Leu Gly Lys Ala Glu Ala Asp Lys Leu Ala Lys
                405                 410                 415

Asp Trp Thr Asn Lys Gly Leu Ser Asn Arg Gln Ile Val Asp Gln Leu
            420                 425                 430

Lys Lys His Phe Ala Ser Thr Gly Asp Thr Ser Ser Asp Asp Ile Leu
        435                 440                 445

Lys Ala Ile Leu Asn Asn Ala Lys Asp Lys Lys Gln Ala Ile Glu Thr
    450                 455                 460

Ile Leu Ala Thr Arg Ile Glu Arg Gln Lys Ala Lys Leu Leu Ala Asp
465                 470                 475                 480

Leu Ile Thr Lys Ile Glu Thr Asp Gln Asn Lys Ile Phe Asn Leu Val
                485                 490                 495

Lys Ser Ala Leu Asn Gly Lys Ala Asp Asp Leu Leu Asn Leu Gln Lys
            500                 505                 510

Arg Leu Asn Gln Thr Lys Lys Asp Ile Asp Tyr Ile Leu Ser Pro Ile
        515                 520                 525

Val Asn Arg Pro Ser Leu Leu Asp Arg Leu Asn Lys Asn Gly Lys Thr
    530                 535                 540

Thr Asp Leu Asn Lys Leu Ala Asn Leu Met Asn Gln Gly Ser Asp Leu
545                 550                 555                 560

Leu Asp Ser Ile Pro Asp Ile Pro Thr Pro Lys Pro Glu Lys Thr Leu
                565                 570                 575

Thr Leu Gly Lys Gly Asn Gly Leu Leu Ser Gly Leu Leu Asn Ala Asp
            580                 585                 590

Gly Asn Val Ser Leu Pro Lys Ala Gly Glu Thr Ile Lys Glu His Trp
        595                 600                 605

Leu Pro Ile Ser Val Ile Val Gly Ala Met Gly Val Leu Met Ile Trp
    610                 615                 620

Leu Ser Arg Arg Asn Lys Leu Lys Asn Lys Ala
625                 630                 635

<210>  28
<211>  635
<212>  PRT
<213>  Staphylococcus aureus

<400>  28

Met Ala Lys Tyr Arg Gly Glu Pro Phe Gln Leu Tyr Val Lys Leu Ser
1               5                   10                  15

Cys Ser Thr Met Met Ala Thr Ser Ile Ile Leu Thr Asn Ile Leu Pro
            20                  25                  30

Tyr Asp Ala Gln Ala Ala Ser Glu Lys Asp Thr Glu Ile Ser Lys Glu
        35                  40                  45

Ile Leu Ser Lys Gln Asp Leu Leu Asp Lys Val Asp Lys Ala Ile Arg
    50                  55                  60

Gln Ile Glu Gln Leu Lys Gln Leu Ser Ala Ser Ser Lys Ala His Tyr
65                  70                  75                  80

Lys Ala Gln Leu Asn Glu Ala Lys Thr Ala Ser Gln Ile Asp Glu Ile
                85                  90                  95

Ile Lys Arg Ala Asn Glu Leu Asp Ser Lys Asp Asn Lys Ser Ser His
            100                 105                 110

Thr Glu Met Asn Gly Gln Ser Asp Ile Asp Ser Lys Leu Asp Gln Leu
        115                 120                 125

Leu Lys Asp Leu Asn Glu Val Ser Ser Asn Val Asp Arg Gly Gln Gln
    130                 135                 140

Ser Gly Glu Asp Asp Leu Asn Ala Met Lys Asn Asp Met Ser Gln Thr
145                 150                 155                 160

Ala Thr Thr Lys His Gly Glu Lys Asp Asp Lys Asn Asp Glu Ala Met
                165                 170                 175

Val Asn Lys Ala Leu Glu Asp Leu Asp His Leu Asn Gln Gln Ile His
            180                 185                 190

Lys Ser Lys Asp Ala Leu Lys Tyr Ala Ser Lys Asp Pro Ala Val Ser
        195                 200                 205

Thr Thr Asp Ser Asn His Glu Val Ala Lys Thr Pro Asn Asn Asp Gly
    210                 215                 220

Ser Gly His Val Val Leu Asn Lys Phe Leu Ser Asn Glu Glu Asn Gln
225                 230                 235                 240

Ser His Ser Asn Arg Leu Thr Asp Lys Leu Gln Gly Ser Asp Lys Ile
                245                 250                 255

Asn His Ala Met Ile Glu Lys Leu Ala Lys Ser Asn Ala Ser Thr Gln
            260                 265                 270

His Tyr Thr Tyr His Lys Leu Asn Thr Leu Gln Ser Leu Asp Gln Arg
        275                 280                 285

Ile Ala Asn Thr Gln Leu Pro Lys Asn Gln Lys Ser Asp Leu Met Ser
    290                 295                 300

Glu Val Asn Lys Thr Lys Glu Arg Ile Lys Ser Gln Arg Asn Ile Ile
305                 310                 315                 320

Leu Glu Glu Leu Ala Arg Thr Asp Asp Lys Lys Tyr Ala Thr Gln Ser
                325                 330                 335

Ile Leu Glu Ser Ile Phe Asn Lys Asp Glu Ala Asp Lys Ile Leu Lys
            340                 345                 350

Asp Ile Arg Val Asp Gly Lys Thr Asp Gln Gln Ile Ala Asp Gln Ile
        355                 360                 365

Thr Arg His Ile Asp Gln Leu Ser Leu Thr Thr Ser Asp Asp Leu Leu
    370                 375                 380

Thr Ser Leu Ile Asp Gln Ser Gln Asp Lys Ser Leu Leu Ile Ser Gln
385                 390                 395                 400

Ile Leu Gln Thr Lys Leu Gly Lys Ala Glu Ala Asp Lys Leu Ala Lys
                405                 410                 415

Asp Trp Thr Asn Lys Gly Leu Ser Asn Arg Gln Ile Val Asp Gln Leu
            420                 425                 430

Lys Lys His Phe Ala Ser Thr Gly Asp Thr Ser Ser Asp Asp Ile Leu
        435                 440                 445

Lys Ala Ile Leu Asn Asn Ala Lys Asp Lys Lys Gln Ala Ile Glu Thr
    450                 455                 460

Ile Leu Ala Thr Arg Ile Glu Arg Gln Lys Ala Lys Leu Leu Ala Asp
465                 470                 475                 480

Leu Ile Thr Lys Ile Glu Thr Asp Gln Asn Lys Ile Phe Asn Leu Val
                485                 490                 495

Lys Ser Ala Leu Asn Gly Lys Ala Asp Asp Leu Leu Asn Leu Gln Lys
            500                 505                 510

Arg Leu Asn Gln Thr Lys Lys Asp Ile Asp Tyr Ile Leu Ser Pro Ile
        515                 520                 525

Val Asn Arg Pro Ser Leu Leu Asp Arg Leu Asn Lys Asn Gly Lys Thr
    530                 535                 540

Thr Asp Leu Asn Lys Leu Ala Asn Leu Met Asn Gln Gly Ser Asn Leu
545                 550                 555                 560

Leu Asp Ser Ile Pro Asp Ile Pro Thr Pro Lys Pro Glu Lys Thr Leu
                565                 570                 575

Thr Leu Gly Lys Gly Asn Gly Leu Leu Ser Gly Leu Leu Asn Ala Asp
            580                 585                 590

Gly Asn Val Ser Leu Pro Lys Ala Gly Glu Thr Ile Lys Glu His Trp
        595                 600                 605

Leu Pro Ile Ser Val Ile Val Gly Ala Met Gly Val Leu Met Ile Trp
    610                 615                 620

Leu Ser Arg Arg Asn Lys Leu Lys Asn Lys Ala
625                 630                 635

<210>  29
<211>  635
<212>  PRT
<213>  Staphylococcus aureus

<400>  29

Met Ala Lys Tyr Arg Gly Lys Pro Phe Gln Leu Tyr Val Lys Leu Ser
1               5                   10                  15

Cys Ser Thr Met Met Ala Ser Ser Ile Ile Leu Thr Asn Ile Leu Pro
            20                  25                  30

Tyr Asp Ala Gln Ala Ala Ser Glu Lys Asp Thr Glu Ile Ser Lys Glu
        35                  40                  45

Ile Leu Ser Lys Gln Asp Leu Leu Asp Lys Val Asp Lys Ala Ile Arg
    50                  55                  60

Gln Ile Glu Gln Leu Lys Gln Leu Ser Ala Ser Ser Lys Ala His Tyr
65                  70                  75                  80

Lys Ala Gln Leu Asn Glu Ala Lys Thr Ala Ser Gln Ile Asp Glu Ile
                85                  90                  95

Ile Lys Arg Ala Asn Glu Leu Asp Ser Lys Glu Asn Lys Ser Ser His
            100                 105                 110

Thr Glu Met Asn Gly Gln Ser Asp Ile Asp Ser Lys Leu Asp Gln Leu

115                 120                 125

Leu Lys Asp Leu Asn Glu Val Ser Ser Asn Val Asp Arg Gly Gln Gln
    130                 135                 140

Ser Gly Glu Asp Asp Leu Asn Ala Met Lys Asn Asp Met Ser Gln Thr
145                 150                 155                 160

Ala Thr Thr Lys Tyr Gly Glu Lys Asp Asp Lys Asn Asp Glu Ala Met
                165                 170                 175

Val Asn Lys Ala Leu Glu Asp Leu Asp His Leu Asn Gln Gln Ile His
            180                 185                 190

Lys Ser Lys Asp Ala Leu Lys Asp Ala Ser Lys Asp Pro Ala Val Ser
        195                 200                 205

Thr Thr Asp Ser Asn His Glu Val Ala Lys Thr Pro Asn Asn Asp Gly
    210                 215                 220

Ser Gly His Val Val Leu Asn Lys Phe Leu Ser Asn Glu Glu Asn Gln
225                 230                 235                 240

Ser His Ser Asn Gln Leu Thr Asp Lys Leu Gln Gly Ser Asp Lys Ile
                245                 250                 255

Asn His Ala Met Ile Glu Lys Leu Ala Lys Ser Asn Ala Ser Thr Gln
            260                 265                 270

His Tyr Thr Tyr His Lys Leu Asn Thr Leu Gln Ser Leu Asp Gln Arg
        275                 280                 285

Ile Ala Asn Thr Gln Leu Pro Lys Asn Gln Lys Ser Asp Leu Met Ser
    290                 295                 300

Glu Val Asn Lys Thr Lys Glu Arg Ile Lys Ser Gln Arg Asn Ile Ile
305                 310                 315                 320

Leu Glu Glu Leu Ala Arg Thr Asp Asp Lys Lys Tyr Ala Thr Gln Ser
                325                 330                 335

Ile Leu Glu Ser Ile Phe Asn Lys Asp Glu Ala Asp Lys Ile Leu Lys
            340                 345                 350

Asp Ile Arg Val Asp Gly Lys Thr Asp Gln Gln Ile Ala Asp Gln Ile
        355                 360                 365

Thr Arg His Ile Asp Gln Leu Ser Leu Thr Thr Ser Asp Asp Leu Leu
    370                 375                 380

Thr Ser Leu Ile Asp Gln Ser Gln Asp Lys Ser Leu Leu Ile Ser Gln

385                 390                 395                 400

Ile Leu Gln Thr Lys Leu Gly Lys Ala Glu Ala Asp Lys Leu Ala Lys
                405                 410                 415

Asp Trp Thr Asn Lys Gly Leu Ser Asn Arg Gln Ile Val Asp Gln Leu
            420                 425                 430

Lys Lys His Phe Ala Ser Thr Gly Asp Thr Ser Ser Asp Asp Ile Leu
        435                 440                 445

Lys Ala Ile Leu Asn Asn Ala Lys Asp Lys Lys Gln Ala Ile Glu Thr
    450                 455                 460

Ile Leu Ala Thr Arg Ile Glu Arg Gln Lys Ala Lys Leu Leu Ala Asp
465                 470                 475                 480

Leu Ile Thr Lys Ile Glu Thr Asp Gln Asn Lys Ile Phe Asn Leu Val
                485                 490                 495

Lys Ser Ala Leu Asn Gly Lys Ala Asp Asp Leu Leu Asn Leu Gln Lys
            500                 505                 510

Arg Leu Asn Gln Thr Lys Lys Asp Ile Asp Tyr Ile Leu Ser Pro Ile
        515                 520                 525

Val Asn Arg Pro Ser Leu Leu Asp Arg Leu Asn Lys Asn Gly Lys Thr
    530                 535                 540

Thr Asp Leu Asn Lys Leu Ala Asn Leu Met Asn Gln Gly Ser Asn Leu
545                 550                 555                 560

Leu Asp Ser Ile Pro Asp Ile Pro Thr Pro Lys Pro Glu Lys Thr Leu
                565                 570                 575

Thr Leu Gly Lys Gly Asn Gly Leu Leu Ser Gly Leu Leu Asn Ala Asp
            580                 585                 590

Gly Asn Val Ser Leu Pro Lys Ala Gly Glu Thr Ile Lys Glu His Trp
        595                 600                 605

Leu Pro Ile Ser Val Ile Val Gly Ala Met Gly Val Leu Met Ile Trp
    610                 615                 620

Leu Ser Arg Arg Asn Lys Leu Lys Asn Lys Ala
625                 630                 635

<210>  30
<211>  631
<212>  PRT
<213>  Staphylococcus aureus

<400> 30

Met Ala Lys Tyr Arg Gly Lys Pro Phe Gln Leu Tyr Val Lys Leu Ser
1               5                   10                  15

Cys Ser Thr Met Met Ala Ile Ser Ile Ile Leu Thr Asn Ile Leu Pro
            20                  25                  30

Tyr Asp Ala Gln Ala Ala Ser Glu Asn Asp Thr Glu Ile Ser Lys Glu
        35                  40                  45

Ile Leu Ser Lys Gln Asp Leu Leu Asp Lys Val Asp Lys Ala Asn Arg
    50                  55                  60

Gln Ile Glu Gln Leu Lys Gln Leu Ser Ala Ser Ser Lys Ala His Tyr
65                  70                  75                  80

Lys Ala Gln Leu Asn Glu Ala Lys Thr Ala Leu Gln Ile Asp Glu Ile
                85                  90                  95

Ile Lys Arg Ala Asn Glu Leu Asp Ser Lys Asp Asn Lys Gly Ser Gln
            100                 105                 110

Ile Glu Leu Asn Gly Glu Ser Asp Ile Asp Ser Lys Leu Asp Gln Leu
        115                 120                 125

Leu Lys Asp Leu Asn Glu Val Ser Ser Lys Val Asp Gly Gly Gln Gln
    130                 135                 140

Ser Gly Glu Asp Asp Leu Asn Ala Met Lys Asn Asp Met Ser Gln Thr
145                 150                 155                 160

Ala Thr Thr Lys His Gly Glu Lys Asp Asp Lys Asn Asp Glu Val Met
                165                 170                 175

Val Asp Lys Ala Leu Glu Asp Leu Asp His Leu Asn Gln Gln Ile Arg
            180                 185                 190

Lys Ser Lys Asp Thr Ser Lys Asp Pro Ala Val Ser Thr Thr Asp Asn
        195                 200                 205

Asn His Glu Val Ala Lys Thr Ser Asn Asn Asp Gly Ser Gly His Val
    210                 215                 220

Val Leu Asn Lys Phe Leu Ser Asn Glu Glu Asn Gln Ser His Ser Asn
225                 230                 235                 240

Arg Leu Thr Asp Lys Leu Gln Gly Ser Asp Lys Ile Asn His Ala Met
                245                 250                 255

Ile Glu Lys Met Ala Lys Ser Asn Ala Ser Thr Gln His Tyr Thr Tyr
            260                 265                 270

His Lys Leu Asn Thr Leu Gln Ser Leu Asp Gln Arg Ile Ala Asn Thr
        275                 280                 285

Gln Leu Pro Lys Asn Gln Lys Ser Asp Leu Met Ser Glu Val Asn Lys
    290                 295                 300

Thr Lys Glu Arg Ile Lys Ser Gln Arg Asn Ile Ile Leu Glu Glu Leu
305                 310                 315                 320

Ala Arg Asn Asn Asp Lys Lys Tyr Ala Thr Gln Ser Ile Leu Glu Ser
                325                 330                 335

Ile Phe Asn Lys Asp Glu Ala Asp Lys Ile Leu Lys Asp Ile Arg Val
            340                 345                 350

Asp Gly Lys Thr Asp Gln Gln Ile Ala Asp Gln Ile Thr Arg His Ile
        355                 360                 365

Asp Gln Leu Ser Leu Thr Thr Ser Asp Asp Leu Leu Thr Ser Leu Ile
    370                 375                 380

Asp Gln Ser Gln Asp Lys Ser Leu Leu Ile Ser Gln Ile Leu Gln Thr
385                 390                 395                 400

Lys Leu Gly Lys Ser Glu Ala Asp Lys Leu Ala Lys Asp Trp Thr Asn
                405                 410                 415

Lys Gly Leu Ser Asn Arg Gln Ile Val Asp Gln Leu Lys Lys Arg Phe
            420                 425                 430

Ala Ser Thr Gly Asp Thr Ser Ser Asp Asp Ile Leu Lys Ala Ile Leu
        435                 440                 445

Asn Asn Ala Lys Asp Lys Lys Gln Ala Ile Glu Thr Ile Leu Ala Thr
    450                 455                 460

Arg Ile Glu Arg Gln Lys Ala Lys Leu Leu Ala Asp Leu Ile Thr Lys
465                 470                 475                 480

Ile Glu Thr Asp Gln Asn Lys Ile Phe Asn Leu Val Lys Ser Ala Leu
                485                 490                 495

Asn Gly Lys Ala Asp Asp Leu Leu Asn Leu Gln Lys Arg Leu Asn Gln
            500                 505                 510

Thr Lys Lys Asp Ile Asp Tyr Val Leu Ser Pro Ile Val Asn Arg Pro
        515                 520                 525

Ser Leu Leu Asp Arg Leu Asn Lys Asn Gly Lys Thr Thr Asp Leu Asn
    530                 535                 540

Lys Leu Ala Asn Leu Met Asn Gln Gly Ser Asn Leu Leu Asp Ser Ile
545                 550                 555                 560

Pro Asp Ile Pro Thr Pro Lys Pro Glu Lys Thr Leu Thr Leu Gly Lys
                565                 570                 575

Gly Asn Gly Leu Leu Ser Gly Leu Leu Asn Ala Asp Gly Asn Val Ser
            580                 585                 590

Leu Pro Lys Ala Gly Glu Thr Ile Lys Glu His Trp Leu Pro Ile Ser
        595                 600                 605

Val Ile Val Gly Ala Met Gly Val Leu Met Ile Trp Leu Ser Arg Arg
    610                 615                 620

Asn Lys Leu Lys Asn Lys Ala
625                 630

<210>  31
<211>  627
<212>  PRT
<213>  Staphylococcus aureus

<400>  31

Met Ala Lys Tyr Arg Gly Lys Pro Phe Gln Leu Tyr Val Lys Leu Ser
1               5                   10                  15

Cys Ser Thr Met Met Ala Thr Ser Ile Ile Leu Thr Asn Ile Leu Pro
            20                  25                  30

Tyr Asp Ala Gln Ala Val Ser Glu Lys Asp Thr Glu Ile Ser Lys Glu
        35                  40                  45

Leu Leu Ser Lys Gln Asp Leu Leu Asp Lys Val Asp Lys Ala Asn Arg
    50                  55                  60

Gln Ile Glu Gln Leu Lys Gln Leu Ser Ala Ser Ser Lys Ala His Tyr
65                  70                  75                  80

Lys Ala Gln Leu Asn Glu Ala Lys Thr Ala Ser Gln Ile Asp Glu Ile
                85                  90                  95

Ile Lys Arg Ala Asn Glu Leu Asp Ser Lys Asp Asn Lys Gly Ser Gln
            100                 105                 110

Ile Glu Met Asn Gly Arg Ser Asp Ile Asp Ser Lys Leu Asp Gln Leu
        115                 120                 125

Leu Lys Asp Leu Asn Glu Val Ser Ser Lys Val Asp Arg Gly Gln Gln
    130                 135                 140

Ser Asp Glu Asp Asp Leu Asn Ala Met Lys Asn Asp Met Ser Gln Thr
145                 150                 155                 160

Ala Thr Thr Lys His Gly Glu Lys Asp Asp Lys Asn Asp Glu Ala Met
                165                 170                 175

Val Asn Lys Ala Leu Glu Asp Leu Asp His Leu Ser Gln Gln Ile His
            180                 185                 190

Lys Ser Glu Asp Ser Ala Val Ser Thr Thr Asp Asn Asn His Glu Val
        195                 200                 205

Ala Lys Thr Pro Asn Asn Asp Gly Ser Gly His Val Val Leu Asn Lys
    210                 215                 220

Phe Leu Ser Asn Glu Glu Asn Gln Ser His Ser Asn Arg Leu Thr Asp
225                 230                 235                 240

Lys Leu Gln Gly Ser Asp Lys Ile Asn His Ala Met Ile Glu Lys Leu
                245                 250                 255

Ala Lys Ser Asn Ala Ser Thr Gln His Tyr Thr Tyr His Lys Leu Asn
            260                 265                 270

Thr Leu Gln Ser Leu Asp Gln Arg Ile Ala Asn Thr Gln Leu Pro Lys
        275                 280                 285

Asn Gln Lys Ser Asp Leu Met Ser Glu Val Asn Lys Thr Lys Glu Arg
    290                 295                 300

Ile Lys Ser Gln Arg Asn Ile Ile Leu Glu Glu Leu Ala Arg Thr Asp
305                 310                 315                 320

Asp Lys Lys His Ala Thr Gln Arg Ile Leu Glu Ser Ile Phe Asn Lys
                325                 330                 335

Asp Glu Ala Asp Lys Ile Leu Lys Asp Ile Arg Val Asp Gly Lys Thr
            340                 345                 350

Asp Gln Gln Ile Ala Asp Gln Ile Thr Arg His Ile Asp Gln Leu Ser
        355                 360                 365

Leu Thr Thr Ser Asp Asp Leu Leu Thr Ser Leu Ile Asp Gln Ser Gln
    370                 375                 380

Asp Lys Ser Leu Leu Ile Ser Gln Ile Leu Gln Thr Lys Leu Gly Lys
385                 390                 395                 400

Ala Glu Ala Asp Lys Leu Ala Lys Asp Trp Thr Asn Lys Gly Leu Ser
                405                 410                 415

Asn Arg Gln Ile Val Asp Gln Leu Lys Lys His Phe Ala Ser Thr Gly
            420                 425                 430

Asp Thr Ser Ser Asp Asp Ile Leu Lys Ala Ile Leu Asn Asn Ala Lys
        435                 440                 445

Asp Lys Lys Gln Ala Ile Glu Thr Ile Leu Ala Thr Arg Ile Glu Arg
    450                 455                 460

Gln Lys Ala Lys Leu Leu Ala Asp Leu Ile Thr Lys Ile Glu Thr Asp
465                 470                 475                 480

Gln Asn Lys Ile Phe Asn Leu Val Lys Ser Ala Leu Asn Gly Lys Ala
                485                 490                 495

Asp Asp Leu Leu Asn Leu Gln Lys Arg Leu Asn Gln Thr Lys Lys Asp
            500                 505                 510

Ile Asp Tyr Val Leu Ser Pro Ile Val Asn Arg Pro Ser Leu Leu Asp
        515                 520                 525

Arg Leu Asn Lys Asn Gly Lys Thr Thr Asp Leu Asn Lys Leu Ala Asn
    530                 535                 540

Leu Met Asn Gln Gly Ser Asn Leu Leu Asp Ser Ile Pro Asp Ile Pro
545                 550                 555                 560

Thr Pro Lys Pro Glu Lys Thr Leu Thr Leu Gly Lys Gly Asn Gly Leu
                565                 570                 575

Leu Ser Gly Leu Leu Asn Ala Asp Gly Asn Val Ser Leu Pro Lys Ala
            580                 585                 590

Gly Glu Thr Ile Lys Glu His Trp Leu Pro Ile Ser Val Ile Val Gly
        595                 600                 605

Ala Met Gly Val Leu Met Ile Trp Leu Ser Arg Arg Asn Lys Leu Lys
    610                 615                 620

Asn Lys Ala
625

<210> 32
<211> 654
<212> PRT
<213> Staphylococcus saprophyticus

<400> 32

Met Lys Lys Leu Arg Tyr Asn Asn Leu Ser Asn Lys Thr Arg Leu Asn
1               5                   10                  15

His Gln Phe Lys Phe Ser Thr Met Ala Leu Ile Ile Ser Thr Ser Leu
            20                  25                  30

Met Gly Tyr Ser Met Thr Ser Thr Pro Ser Val Glu Ala Lys Asp Lys
        35                  40                  45

Ala Asp Leu Asn Ile Glu Thr His Ser Asn Ser Glu Asn Thr Leu Glu
    50                  55                  60

Lys Arg Ile Gln Glu Gly Lys Glu Lys Ile Asp Lys Leu Lys Asn Ile
65                  70                  75                  80

Lys Asp Ser Gln Lys Asp Ala Ser Ile Lys Glu Ile Thr Lys Ala Lys
                85                  90                  95

Ser Thr Glu Glu Val Glu Ala Ile Leu Lys Lys Ala Lys Lys Val Asp
            100                 105                 110

Asn Lys Ile Ile Glu Gln Asn Arg Val Gln Ser His Leu Ile Glu Asn
        115                 120                 125

Asp Lys Lys Glu Val Ser Glu Asp Lys Lys Ser Val Lys Ser Glu Leu
    130                 135                 140

Asp Ser Lys Lys Asn Ile Val Ser Ser Val Lys Glu Lys Ser Asn His
145                 150                 155                 160

Ile Glu Glu Gln Asp Ser Ile Asp Leu Phe Asp Gln Glu Asp Leu Gln
                165                 170                 175

Asp Asn Thr Phe Asp Ala Asn Leu Asn Gln Arg Asp Thr Lys Gln Asp
            180                 185                 190

Ile Ser His Leu Val Asp Met Gly Lys Leu Asn Glu Glu Ser Lys Asp
        195                 200                 205

Ile Ala Asp Met Asn Asp Ser Glu Glu Asn Lys Asn Thr Ala Ser Glu
    210                 215                 220

Lys Asp Glu Gly Ala Val Lys Glu Asn Asn Gln Asp Gly Leu Ala Phe
225                 230                 235                 240

Lys Asp Thr Ser Asn Thr Lys Ser Asn Gln Met Gln Gln Ser Asp Glu
                245                 250                 255

Ile Lys Lys Asp Ile Asp Lys Val Thr Thr Asn His Ser Lys Val Lys
            260                 265                 270

Asp Asn Leu Asp Tyr Tyr Val Glu Asn Lys Glu Asn Asn Leu Lys Ile
        275                 280                 285

Leu Asp Ser Lys Leu Ser Glu Arg Asp Ser Ile Ser Ala Lys Asn Lys
    290                 295                 300

Glu Lys Leu Lys Lys Glu Ile Glu Lys Thr Gln Gln Ser Leu Lys Lys
305                 310                 315                 320

Gln Asn Asp Val Val Leu Asn His Leu Gln Ser Val Asn Asn Lys Glu
                325                 330                 335

Gln Ala Val Lys Asp Ile Val Ser Gly Thr Phe Asp Glu Lys Ser Ala
            340                 345                 350

Gln Ser Ile Leu Glu Arg Ile Asp Thr Lys Gly Lys Thr Asp Gln Gln
        355                 360                 365

Ile Ala Ser Gln Val Val Ser Glu Leu Asp Asn Leu Ser Thr Thr Thr
    370                 375                 380

Ser Asp Asp Ile Leu Lys Ser Met Phe Asp Lys Thr Ser Asp Lys Gln
385                 390                 395                 400

Glu Leu Ile Lys Thr Ile Leu Leu Thr Lys Phe Asp His Ile Asp Thr
                405                 410                 415

Ser Lys Ile Val Asp Glu Ile Met His Lys Asn Pro Ser Asn Glu Gln
            420                 425                 430

Ile Val Ala Leu Ile Lys His His Phe Gly Asp Asn Val Thr Ser Asp
        435                 440                 445

Asp Ile Leu Glu Asn Ile Leu Asp Gln Ser His Asp Lys Arg Lys Ala
    450                 455                 460

Leu Glu Thr Met Leu Ala Thr Lys Leu Asn Asp Ala Lys Ala Lys Ala
465                 470                 475                 480

Leu Ala Asp Val Ile Ala Lys Lys Glu Asp Ser Lys His Asn Leu Leu
                485                 490                 495

Asn Leu Met Lys Ser Gly Ile Asn Asn Glu Leu Asn Asp Leu Leu Lys
            500                 505                 510

Ala Asp Lys Asp Ile Ser Lys Phe Lys Asp Asp Met His Gly Leu Phe
        515                 520                 525

Glu Pro Leu Lys Tyr Thr Pro Ser Leu Ser Asn Lys Phe Asp Gly Ser
    530                 535                 540

Leu Leu Asp Arg Ala Glu Gln Met Arg Lys Leu Ser Gly Asn Ser Lys
545                 550                 555                 560

Leu Leu Gly Thr Pro Ser Leu Phe Asp Asp Leu Phe Asn Arg Asn Ser
                565                 570                 575

Ile Leu Asp Gly Ile Lys Asp Ile Ser Asn Pro Ser Pro Gly Arg Ala
            580                 585                 590

Leu Ser Leu Gly Asp Ser Ser Gly Ser Phe Leu Ser Gly Leu Phe Asp
        595                 600                 605

Asn Asn Gly Asp Phe Ser Leu Pro Asp Thr Gly Thr Val Val Lys Lys
    610                 615                 620

Ser Thr Ile Pro Leu Gly Ile Leu Leu Phe Ile Ile Gly Gly Gly Leu
625                 630                 635                 640

Ile Trp Phe Ile Lys Arg Asn Lys Ser Lys Asn Cys Lys Tyr
                645                 650